# EUROPEAN PATENT SPECIFICATION

(11) **EP 1 460 945 B1**
(45) Date of publication and mention of the grant of the patent: **09.01.2013**
(21) Application number: 02773432.6
(22) Date of filing: 16.09.2002
(51) Int. Cl.: A61B 18/14

(54) **ELECTROSURGICAL APPARATUS FOR TISSUE TREATMENT & REMOVAL**
ELEKTROCHIRURGISCHES GERÄT FÜR DIE GEWEBEBEHANDLUNG UND ENTFERNUNG
APPAREIL ELECTROCHIRURGICAL ASSOCIES PERMETTANT L'EXTRACTION ET LE TRAITEMENT D'UN TISSU

(30) Priority: 14.09.2001 US 322243 P
(43) Date of publication of application: 29.09.2004
(73) Proprietor: ArthroCare Corporation, Austin, TX 78735 (US)
(72) Inventor: DAHLA, Robert, H., Sunnyvale, CA 94086 (US); WOLOSZKO, Jean, Mountain View, CA 94040 (US)
(74) Representative: Warren, Caroline Elisabeth
(86) International application number: PCT/US2002/029476
(87) International publication number: WO 2003/024305

(56) References cited:
- WO-A-01/24720
- WO-A1-01/95819
- US-A- 5 374 261
- US-A- 5 697 882
- US-A- 5 697 882
- US-B1- 6 254 600

## Description

### BACKGROUND OF THE INVENTION

The present invention relates generally to the field of electrosurgery and, more particularly, to surgical devices and methods which employ high frequency voltage to cut, abate, treat, or modify body tissue. This invention also relates to an electrode terminal for an electrosurgical apparatus, the electrode terminal having an electrode lumen coupled to a vacuum source. This invention further relates to an electrode assembly for an electrosurgical probe, the electrode assembly including an electrode support having a suction cavity therein. This invention still further relates to an electrosurgical probe having an integral aspiration unit, the aspiration unit comprising an electrode lumen within an electrode terminal of the probe.

Conventional electrosurgical methods are widely used since they generally reduce patient bleeding associated with tissue cutting operations and improve the surgeon's visibility. These traditional electrosurgical techniques for treatment have typically relied on thermal methods to rapidly heat and vaporize liquid within tissue and to cause cellular destruction. In conventional monopolar electrosurgery, for example, electric current is directed along a defined path from the exposed or active electrode through the patient's body to the return electrode, which is externally attached to a suitable location on the patient's skin. In addition, since the defined path through the patient's body has a relatively high electrical impedance, large voltage differences must typically be applied between the active and return electrodes to generate a current suitable for cutting or coagulation of the target tissue. This current, however, may inadvertently flow along localized pathways in the body having less impedance than the defined electrical path. This situation will substantially increase the current flowing through these paths, possibly causing damage to or destroying tissue along and surrounding this pathway.

Bipolar electrosurgical devices have an inherent advantage over monopolar devices because the return current path does not flow through the patient beyond the immediate site of application of the bipolar electrodes. In bipolar devices, both the active and return electrode are typically exposed so that they may both contact tissue, thereby providing a return current path from the active to the return electrode through the tissue. One drawback with this configuration, however, is that the return electrode may cause tissue desiccation or destruction at its contact point with the patient's tissue.

Another limitation of conventional bipolar and monopolar electrosurgery devices is that they are not suitable for the precise removal (i.e., ablation) of tissue. For example, conventional electrosurgical cutting devices typically operate by creating a voltage difference between the active electrode and the target tissue, causing an electrical arc to form across the physical gap between the electrode and tissue. At the point of contact of the electric arcs with tissue, rapid tissue heating occurs due to high current density between the electrode and tissue. This high current density causes cellular fluids to rapidly vaporize into steam, thereby producing a "cutting effect" along the pathway of localized tissue heating. The tissue is parted along the pathway of evaporated cellular fluid, inducing undesirable collateral tissue damage in regions surrounding the target tissue site.

The use of electrosurgical procedures (both monopolar and bipolar) in electrically conductive environments can be further problematic. For example, many arthroscopic procedures require flushing of the region to be treated with isotonic saline, both to maintain an isotonic environment and to keep the field of view clear. However, the presence of saline, which is a highly conductive electrolyte, can cause shorting of the active electrode(s) in conventional monopolar and bipolar electrosurgery. Such shorting causes unnecessary heating in the treatment environment and can further cause non-specific tissue destruction.

Conventional electrosurgical techniques used for tissue ablation also suffer from an inability to control the depth of necrosis in the tissue being treated. Most electrosurgical devices rely on creation of an electric arc between the treating electrode and the tissue being cut or ablated to cause the desired localized heating. Such arcs, however, often create very high temperatures causing a depth of necrosis greater than 500 µm, frequently greater than 800 µm, and sometimes as great as 1700 µm. The inability to control such depth of necrosis is a significant disadvantage in using electrosurgical techniques for tissue ablation, particularly in arthroscopic procedures for ablating and/or reshaping fibrocartilage, articular cartilage, meniscal tissue, and the like.

In an effort to overcome at least some of these limitations of electrosurgery, laser apparatus have been developed for use in arthroscopic and other surgical procedures. Lasers do not suffer from electrical shorting in conductive environments, and certain types of lasers allow for very controlled cutting with limited depth of necrosis. Despite these advantages, laser devices suffer from their own set of deficiencies. In the first place, laser equipment can be very expensive because of the costs associated with the laser light sources. Moreover, those lasers which permit acceptable depths of necrosis (such as excimer lasers, erbium:YAG lasers, and the like) provide a very low volumetric ablation rate, which is a particular disadvantage in cutting and ablation of fibrocartilage, articular cartilage, and meniscal tissue. The holmium:YAG and Nd:YAG lasers provide much higher volumetric ablation rates, but are much less able to control depth of necrosis than are the slower laser devices. The CO₂ lasers provide high rate of ablation and low depth of tissue necrosis, but cannot operate in a liquid-filled cavity.

Excimer lasers, which operate in an ultraviolet wavelength, cause photo-dissociation of human tissue, commonly referred to as cold ablation. Through this mechanism, organic molecules can be disintegrated into light hydrocarbon gases that are removed from the target site. Such photo-dissociation reduces the likelihood of thermal damage to tissue outside of the target site. Although promising, excimer lasers must be operated in pulses so that ablation plumes created during operation can clear. This prevents excessive secondary heating of the plume of ablation products which can increase the likelihood of collateral tissue damage aswell as a decrease in the rate of ablation. Unfortunately, the pulsed mode of operation reduces the volumetric ablation rate, which may increase the time spent in surgery.

Thus there is a need for apparatus and methods for effecting the controlled ablation, coagulation, or other modification of a target tissue *in vivo,* at a relatively low cost, and with no or minimal collateral tissue damage. The present invention provides such apparatus, as is described in enabling detail hereinbelow.

International Patent Application No. WO01/24720 describes an electrosurgical aspiration instrument that permits aspiration of an area being treated by the instrument. The instrument includes an energy application surface area at a distal end. An aspiration lumen is formed through the instrument with an opening through the energy application surface area which is configured to reduce blockage of the opening. The first part of claim 1 is based on this document.

United States Patent No. US5,697,882 describes an electrosurgical probe having an electrode array at its distal end and a connector at its proximal end for coupling the electrode array to a high frequency power supply. The return electrode defines an inner passage electrically connected to both the return electrode and the electrode array for passage of an electrically conducting liquid.

United States Patent No. US6,254,600 describes systems and methods for selectively applying electrical energy to a target location within or on a patient's body.

International Application Publication Number WO01/95819 A1 describes electrosurgical methods, systems, and apparatus for the controlled ablation of tissue from a target site of a patient. An electrosurgical instrument includes a working portion having a plurality of working zones differentiated according to their relative rates of aspiration and ablation. The ablation by-products may be aspired from the target site via aspiration port(s) on one or more of the plurality of working zones.

Aspects and examples of the invention are set out in the claims.

The present invention provides systems and apparatus for selectively applying electrical energy to body tissue. The present invention further provides systems and apparatus for electrosurgical treatment or removal of a target tissue of a patient. Described herein is a method which comprises positioning an electrosurgical probe or catheter adjacent the target site so that one or more active electrode(s) are brought into contact with, or close proximity to, a target tissue in the presence of electrically conductive fluid. The electrically conductive fluid may be delivered directly to the active electrode(s) and the target tissue, or the entire target site may be submersed within the conductive fluid. High frequency voltage is then applied between the electrode terminal(s) and one or more return electrode(s) to generate a plasma adjacent to the active electrode(s), and to volumetrically remove or ablate at least a portion of the target tissue. The high frequency voltage generates electric fields around the active electrodes) with sufficient energy to ionize the conductive fluid adjacent to the active electrode(s). Within the ionized gas or plasma, free electrons are accelerated, and electron-atoms collisions liberate more electrons, and the process cascades until the plasma contains sufficient energy to break apart the tissue molecules, causing molecular dissociation and ablation of the target tissue.

In some embodiments, the high frequency voltage applied to the electrode terminal(s) is sufficient to vaporize the electrically conductive fluid (e.g., gel or saline) between the electrode terminal(s) and the tissue. Within the vaporized fluid, a plasma is formed and charged particles (e.g., electrons) cause the molecular dissociation of several cell layers of the tissue. This molecular dissociation is accompanied by the volumetric removal of the tissue. This process can be precisely controlled to effect the volumetric removal of tissue as thin as 10 to 150 microns with minimal heating of, or damage to, surrounding or underlying tissue structures. A more complete description of this phenomenon is described in commonly assigned U.S. Patent No. 5,697,882.

In some embodiments, the tissue is ablated by directly contacting the target tissue with the plasma. In other embodiments, the active electrode(s) are spaced from the tissue a sufficient distance to minimize or avoid contact between the tissue and the plasma formed around the active electrode(s). Applicant believes that the electrons that carry the electrical current contain more thermal energy than the ions within the plasma. In these embodiments, contact between the heated electrons in the plasma and the tissue is minimized as these electrons travel from the plasma back through the conductive fluid to the return electrode(s). The ions within the plasma will have sufficient energy, however, under certain conditions such as higher voltages, to accelerate beyond the plasma to the tissue. Thus, the electrons, which are carried away from the target tissue, carry most of the thermal byproducts of the plasma with them, allowing the ions to break apart the tissue molecules in a substantially non-thermal manner.

Described herein is apparatus which generally includes an electrosurgical instrument having a shaft with proximal and distal ends, one or more active electrode(s) at the distal end and one or more connectors coupling the active electrode(s) to a source of high frequency electrical energy. In some embodiments, the instrument will comprise a catheter designed for percutaneous and/or transluminal delivery. In other embodiments, the instrument will comprise a more rigid probe designed for percutaneous or direct delivery in either open procedures or port access type procedures. In both embodiments, the apparatus will include a high frequency power supply for applying a high frequency voltage to the electrode terminal(s).

The apparatus may further include a supply or source of an electrically conductive fluid, and a fluid delivery element for delivering electrically conductive fluid to the electrode terminal(s) and the target site. The fluid delivery element may be located on the instrument, e.g., a fluid lumen or tube, or it may be part of a separate instrument. Alternatively, an electrically conductive gel or spray may be applied to the target site. In this embodiment, the apparatus may not have a fluid delivery element. In both embodiments, the electrically conductive fluid will preferably generate a current flow path between the active electrode(s) and one or more return electrode(s). In one embodiment, the return electrode is spaced a sufficient distance from the active electrode(s) to substantially avoid or minimize current shorting therebetween, and to shield the tissue at the target site from the return electrode.

The electrosurgical instrument may include an electrically insulating electrode support member or spacer, preferably an inorganic support material (e.g., ceramic, glass, glass/ceramic, etc.). The spacer separates the electrode terminals) from the return electrode. In one embodiment, the instrument includes an electrode array having a plurality of electrically isolated electrode terminals extending about 0.0 mm to about 10 mm distally from the distal end of the instrument. The probe may further include one or more lumens for delivering electrically conductive fluid and/or aspirating the target site.

In one configuration, the instrument includes a fluid delivery lumen for delivering electrically conductive fluid to the active and/or return electrodes, and an aspiration lumen for aspirating excess conductive fluid from the distal end of the apparatus or from the surgical site. In one embodiment, the fluid delivery and aspiration lumens create a fluid recirculation system for minimizing the amount of conductive fluid that contacts the patient, and for reducing the temperature to which a target tissue is exposed during a procedure.

Also described herein is an electrosurgical probe including an electrode assembly having at least one active electrode terminal, each electrode terminal arranged in an electrode socket of an electrically insulating electrode support. The electrode support includes a suction cavity coupled proximally to an aspiration lumen, and each active electrode terminal has an electrode lumen and a suction opening in communication with the suction cavity, and the electrode lumen defines a portion of an aspiration stream. Unwanted materials, such as ablation by-products, may be conveniently removed from a surgical site during use of the probe in the aspiration stream.

In another embodiment, the invention provides an electrosurgical probe comprising an electrode assembly including an electrically insulating electrode support having a treatment surface, and at least one active electrode terminal, each active electrode terminal having a working end, and the working end protruding from the treatment surface. Typically, the probe further includes a shaft, the electrode assembly disposed at the shaft distal end, and a handle affixed to the shaft proximal end, the handle housing a connection block. In one embodiment, the shaft comprises a metal tube having a proximal portion encased within an electrically insulating sleeve, and an exposed distal portion defining a return electrode. The connection block is adapted for coupling the active electrode terminal(s) and the return electrode to a high frequency power supply.

According to another embodiment, there is provided an active electrode assembly including a plurality of active electrode terminals mounted within an electrically insulating electrode support, wherein a working end of each active electrode terminal protrudes from a treatment surface of the electrode support. In one embodiment, each active electrode terminal has a substantially cylindrical body and a suction opening located at a first region of the working end, the suction opening defining a region of relatively high flow rate of an aspiration stream adjacent to the suction opening, and the working end having a second, shielded region characterized by a relatively low flow rate of the aspiration stream, wherein the second region is located substantially opposite the suction opening. The relatively high flow rate of the first region tends to shield the second region from the effects of the aspiration stream, thereby promoting the generation and maintenance of a plasma at the working end of each electrode terminal upon application of a suitable high frequency voltage to the electrode terminals.

Described herein is a method for ablating or modifying a target tissue at a surgical site, wherein the method involves positioning an electrode assembly of an electrosurgical probe in at least close proximity to the target tissue, and delivering an electrically conductive fluid to the electrode assembly. The electrode assembly includes an active electrode terminal disposed on an electrically insulating electrode support, the electrode terminal has an electrode lumen therethrough, and the electrode lumen defines a portion of an aspiration stream for removing unwanted materials from the surgical site. The method further involves applying a high frequency voltage between the active electrode terminal and a return electrode, wherein the high frequency voltage is sufficient to ablate, coagulate, or otherwise modify the target tissue. The method still further involves aspirating excess electrically conductive fluid, or other unwanted materials (such as gaseous ablation by-products, and the like) from the surgical site. In one embodiment, such unwanted materials are removed from the surgical site in an aspiration stream running through the electrode lumen of the active electrode terminal to a proximal aspiration lumen via a suction cavity of the electrode support. The aspiration lumen may be coupled to an aspiration tube, and the aspiration tube may, in turn, be coupled to a suitable vacuum source.

For a further understanding of the nature and advantages of the invention, reference should be made to the following description taken in conjunction with the accompanying drawings.

### BRIEF DESCRIPTION OF THE DRAWINGS

Fig. 1 is a perspective view of an electrosurgical system incorporating a power supply and an electrosurgical probe;
Fig. 2 schematically illustrates one embodiment of a power supply;
Fig. 3 illustrates an electrosurgical system incorporating a plurality of active electrodes and associated current limiting elements;
Fig. 4 is a side view of an electrosurgical probe;
Figs. 5-8 illustrates an alternative probe incorporating an aspiration lumen;
Fig. 9 illustrates a method of ablating tissue with a probe having a plurality of active electrodes;
Fig. 10 illustrates a method of ablating tissue with a probe having a single active electrode;
Fig. 11 is a perspective view of another electrosurgical system incorporating a power supply, an electrosurgical probe, and a source of electrically conductive fluid;
Fig. 12 is a perspective view of an electrosurgical catheter system, according to the present invention;
Fig. 13 is a block diagram schematically representing an electrosurgical system;
Fig. 14 is a block diagram schematically representing an electrosurgical probe;
Fig. 15 is a block diagram schematically representing an aspiration unit for an electrosurgical apparatus;
Fig. 16 is a block diagram schematically representing an electrode assembly;
Fig. 17A is a perspective view, and Fig. 17B is a longitudinal sectional view, of a distal end of an electrosurgical plobe;
Fig. 18A is side view of an electrosurgica probe;
Fig. 18B shows an electrode assembly of the probe of Fig. 18A;
Fig. 19 is a side view of an electrosurgical probe having an integral aspiration unit and a fluid delivery unit;
Fig. 20 is a perspective view of an active electrode terminal;
Fig. 21A is a perspective view of an active electrode terminal;
Figs. 21B-D illustrates the active electrode terminal of Fig. 21A as taken along the lines 21B-21B, 21C-21C, and 21D-21D, respectively;
Fig. 22 is a perspective view of an electrode support showing a suction cavity and a plurality of intersecting electrode sockets;
Fig. 23A is a perspective view of an electrode assembly;
Fig. 23B is a side view of the electrode assembly of Fig. 23A; and
Fig. 24 schematically represents a number of steps involved in a method of treating a target tissue of a patient using an electrosurgical probe.

### DESCRIPTION OF SPECIFIC EMBODIMENTS

In the present invention, high frequency (RF) electrical energy is applied to one or more electrode terminals in the presence of electrically conductive fluid to remove and/or modify body tissue. The techniques of the present invention may be performed in a conventional open surgery environment or in a minimally invasive manner using cannulas, or port access devices. The present invention is useful in procedures where the tissue site is flooded or submerged with an electrically conductive fluid, such as arthroscopic surgery of the knee, shoulder, ankle, hip, elbow, hand or foot. Specifically, the present invention is useful in the resection and/or ablation of the meniscus and the synovial tissue within a joint during an arthroscopic procedure. In addition, tissues which may be treated by the system and method of the present invention include, but are not limited to, prostate tissue and leiomyomas (fibroids) located within the uterus, gingival tissues and mucosal tissues located in the mouth, tumors, scar tissue, myocardial tissue, collagenous tissue within the eye, or epidermal and dermal tissues on the surface of the skin. The present invention is also useful for resecting tissue within accessible sites of the body that are suitable for electrode loop resection, such as the resection of prostate tissue, leiomyomas (fibroids) located within the uterus, and other diseased tissue within the body.

The present invention is also useful for treating tissue in the head and neck, such as the ear, mouth, pharynx, larynx, esophagus, nasal cavity and sinuses. The head and neck procedures may be performed through the mouth or nose using speculae or gags, or using endoscopic techniques, such as functional endoscopic sinus surgery (FESS). These procedures may include the removal of swollen tissue, chronically-diseased inflamed and hypertrophic mucus linings, polyps, turbinates and/or neoplasms from the various anatomical sinuses of the skull, the turbinates and nasal passages, in the tonsil, adenoid, epi-glottic and supra-glottic regions, and salivary glands, submucous resection of the nasal septum, excision of diseased tissue and the like. In other procedures, the present invention may be useful for collagen shrinkage, ablation and/or hemostasis in procedures for treating swollen tissue (e.g., turbinates) or snoring and obstructive sleep apnea (e.g., soft palate, such as the uvula, or tongue/pharynx stiffening, and midline glossectomies), for gross tissue removal, such as tonsillectomies, adenoidectomies, tracheal stenosis and vocal cord polyps and lesions, or for the resection or ablation of brain tumors, facial tumors, or tumors within the mouth and pharynx. In addition, the present invention is useful for procedures within the ear, such as stapedotomies, tympanostomies or the like.

The present invention may also be useful for treating tissue of the brain and vertebral column. These procedures include tumor removal, laminectomy/disketomy procedures for treating herniated disks, decompressive laminectomy for stenosis in the lumbosacral and cervical spine, medial facetectomy, posterior lumbosacral and cervical spine fusions, treatment of scoliosis associated with vertebral disease, foraminotomies to remove the roof of the intervertebral foramina to relieve nerve root compression and anterior cervical and lumbar diskectomies. These procedures may be performed through open procedures, or using minimally invasive techniques, such as thoracoscopy, arthroscopy, laparascopy or the like.

The present invention may also be useful for cosmetic and plastic surgery procedures in the head and neck. For example, the present invention is useful for ablation and sculpting cartilage tissue, such as the cartilage within the nose that is sculpted during rhinoplasty procedures. The present invention may also be employed for skin tissue removal and/or collagen shrinkage in the epidermis or dermis tissue in the head and neck, e.g., the removal of pigmentations, vascular lesions (e.g., leg veins), scars, tattoos, etc., and for other surgical procedures on the skin, such as tissue rejuvenation, cosmetic eye procedures (blepharoplasties), wrinkle removal, tightening muscles for facelifts or browlifts, hair removal and/or transplant procedures, etc.

The systems, apparatus, and methods of the invention are applicable to a broad range of procedures including, without limitation: open procedures, intravascular procedures, interventional cardiology procedures, urology, laparascopy, arthroscopy, thoracoscopic or other cardiac procedures, cosmetic surgery, orthopedics, gynecology, otorhinolaryngology, spinal and neurologic procedures, oncology and the like.

In one aspect of the invention, the body tissue is volumetrically removed or ablated. In this procedure, a high frequency voltage difference is applied between one or more electrode terminal(s) and one or more return electrode(s) to develop high electric field intensities in the vicinity of the target tissue. The high electric field intensities adjacent the electrode terminal(s) lead to electric field induced molecular breakdown of target tissue via molecular dissociation (as opposed to thermal evaporation or carbonization). Applicant believes that the tissue structure is volumetrically removed through molecular disintegration of larger organic molecules into smaller molecules and/or atoms, such as hydrogen, oxygen, oxides of carbon, hydrocarbons and nitrogen compounds. This molecular disintegration completely removes the tissue structure, as opposed to dehydrating the tissue material by the removal of liquid within the cells of the tissue, as is typically the case with electrosurgical desiccation.

The high electric field intensities may be generated by applying a high frequency voltage that is sufficient to vaporize an electrically conductive fluid over at least a portion of the electrode terminal(s) in the region between the distal tip of the electrode terminal(s) and the target tissue. The electrically conductive fluid may be a liquid, such as isotonic saline or blood, delivered to the target site, a viscous fluid, such as a gel, or a gas. Since the vapor layer or vaporized region has a relatively high electrical impedance, it minimizes the current flow into the electrically conductive fluid). This ionization, under the conditions described herein, induces the discharge of energetic electrons and photons from the vapor layer and to the surface of the target tissue. A more detailed description of this process, termed Coblation^{®} can be found in commonly assigned U.S. Patent No. 5,697, 882.

Applicant believes that the principal mechanism of tissue removal in the Coblation™ process of the present invention is via the action of charged particles (e.g., energetic electrons) that have been energized in a plasma adjacent to the electrode terminal(s). When a liquid is heated enough that atoms vaporize off the surface faster than they recondense, a gas is formed. When the gas is heated enough that the atoms collide with each other and knock their electrons off in the process, an ionized gas or plasma is formed (the so-called "fourth state of matter"). A more complete description of plasma can be found in Plasma Physics, by R.J. Goldston and P.H. Rutherford of the Plasma Physics Laboratory of Princeton University (1995). When the density of the vapor layer (or within a bubble formed in the electrically conductive liquid) becomes sufficiently low (i.e., less than approximately 10²⁰ atoms/cm³ for aqueous solutions), the electron mean free path increases to enable subsequently injected electrons to cause impact ionization within these regions of low density (i.e., vapor layers or bubbles). Once the ionic particles in the plasma layer have sufficient energy, they accelerate towards the target tissue. Energy evolved by the energetic electrons (e.g., 3.5 eV to 5 eV) can subsequently bombard a molecule and break its bonds, dissociating a molecule into free radicals, which then combine into final gaseous or liquid species.

Plasmas may be formed by heating a small volume of gas and ionizing the gas by driving an electric current through it, or by transmitting radio waves into the gas. Generally, these methods of plasma formation give energy to free electrons in the plasma directly, and then electron-atom collisions liberate more electrons, and the process cascades until the desired degree of ionization is achieved. Often, the electrons carry the electrical current or absorb the radio waves and, therefore, are hotter than the ions. Thus, in applicant's invention, the electrons, which are carried away from the tissue towards the return electrode, carry most of the plasma's heat with them, allowing the ions to break apart the tissue molecules in a substantially non-thermal manner.

Applicant has found that increasing the current densities around the electrode terminal(s) can lead to higher energy levels in the ionized plasma. This, in turn, allows the ionized plasma to break stronger molecular bonds, such as those present in bone or calcified fragments. Since the electrically conductive fluid between the target site and electrode terminal(s) is transformed into an ionized vapor layer or plasma, the number of charged particles which can be accelerated against the target also determines the ablation rate. In addition, the conductivity of the fluid may have an effect on the strength of the plasma field created at the end of the probe. Typically, isotonic saline with a concentration of 0.9 % sodium chloride is used with the probe. In some embodiments, increasing the sodium chloride concentration to greater than 0.9%, and perhaps between about 3% and 20 % , may lead to increased rates of tissue ablation. This concept of using a hypertonic saline solution with enhanced conductivity and increased numbers of charged particles is of particular use in bone removal processes or in other procedures requiring aggressive volumetric removal.

Applicant has also found that the plasma layer typically requires a higher voltage level to initiate a plasma than to sustain the plasma once it has been initiated. In addition, it has been found that some conductive solutions facilitate the initiation of the plasma layer, rather than the energy level of the plasma, as discussed above. For example, it has been found that saline solutions having concentrations less than isotonic saline (i.e., less than 0.9 % sodium chloride) facilitate the initiation of the plasma layer. This may be useful in applications where initiation of the plasma layer is more difficult, such as applications where a suction pressure is applied near the electrode terminals). A more complete description of this type of application, and the devices that carry out simultaneous suction and ablation can be found in U.S. Patent Application No. 09/010,382, filed January 21, 1998 (Attorney Docket No. A-6).

In some embodiments, the present invention applies high frequency (RF) electrical energy in an electrically conductive fluid environment to remove (i.e., resect, cut or ablate) a tissue structure and to seal transected vessels within the region of the target tissue. The present invention is particularly useful for sealing larger arterial vessels, e.g., on the order of 1 mm or greater. In some embodiments, a high frequency power supply is provided having an ablation mode, wherein a first voltage is applied to an electrode terminal sufficient to effect molecular dissociation or disintegration of the tissue, and a sub-ablation mode, wherein a second, lower voltage is applied to an electrode terminal (either the same or a different electrode) sufficient to achieve non-ablative treatment of tissue, e.g., shrinkage of tissue, coagulation or hemostasis of severed vessels within the tissue. In other embodiments, an electrosurgical instrument is provided having one or more coagulation electrode(s) configured for sealing a severed vessel, such as an arterial vessel, and one or more electrode terminals configured for either contracting the collagen fibers within the tissue, or removing (ablating) the tissue, e.g., by applying sufficient energy to the tissue to effect molecular dissociation. In the latter embodiments, the coagulation electrode(s) may be configured such that a single voltage can be applied to coagulate with the coagulation electrods(s), and to ablate with the electrode terminal(s). In other embodiments, the power supply is combined with the electrosurgical instrument such that the coagulation electrode is used when the power supply is in the coagulation mode (low voltage), and the electrode terminal(s) are used when the power supply is in the ablation mode (higher voltage). In other embodiments, an electrosurgical instrument is provided having a single active electrode adapted for cutting, volumetrically removing, shrinking, and coagulating a target tissue. In these embodiments, the nature of the tissue treatment or effect depends on, among other factors, the geometry and configuration of the active and return electrodes, the type of tissue at the treatment site, the manner in which the active electrode is manipulated with respect to the tissue, and/or the voltage applied from the power supply (e.g., in the ablation mode or the sub-ablation mode). Manipulation of an electrosurgical probe, to effect different types of tissue treatment are discussed hereinbelow.

In one method, one or more electrode terminals are brought into at least close proximity to a target tissue, and the power supply is activated in the ablation mode such that sufficient voltage is applied between the electrode terminals and the return electrode to volumetrically remove the tissue through molecular dissociation, as described below. During this process, vessels within the tissue will be severed. Smaller vessels will be automatically sealed with the system and method of the present invention. Larger vessels, and those with a higher flow rate, such as arterial vessels, may not be automatically sealed in the ablation mode. In these cases, the severed vessels may be sealed by activating a control (e.g., a foot pedal) to reduce the voltage of the power supply into the coagulation (sub-ablation) mode. In the sub-ablation mode, the electrode terminals may be pressed against the severed vessel to provide sealing and/or coagulation of the vessel. Alternatively, a coagulation electrode located on the same or a different instrument may be pressed against the severed vessel. Once the vessel is adequately sealed, the surgeon activates a control (e.g., another foot pedal) to increase the voltage of the power supply back into the ablation mode.

The present invention is also useful for removing or ablating tissue around nerves, such as spinal, or cranial nerves, e.g., optic nerve, facial nerves, vestibulocochlear nerves and the like. One of the significant drawbacks with the prior art microdebriders and lasers is that these devices do not differentiate between the target tissue and the surrounding nerves or bone. Therefore, the surgeon must be extremely careful during these procedures to avoid damage to the bone or nerves around the target site. In the present invention, the Coblation^{®} process for removing tissue results in extremely small depths of collateral tissue damage as discussed above. This allows the surgeon to remove tissue close to a nerve without causing collateral damage to the nerve fibers.

In addition to the generally precise nature of the novel mechanisms of the present invention, applicant has discovered an additional method of ensuring that adjacent nerves are not damaged during tissue removal. According to the present invention, systems and methods are provided for distinguishing between the fatty tissue immediately surrounding nerve fibers and the normal tissue that is to be removed during the procedure. Peripheral nerves usually comprise a connective tissue sheath, or epineurium, enclosing the bundles of nerve fibers, each bundle being surrounded by its own sheath of connective tissue (the perineurium) to protect these nerve fibers. The outer protective tissue sheath or epineurium typically comprises a fatty material having substantially different electrical properties than "normal" target tissue. The system of the present invention measures the electrical properties of the tissue at the tip of the probe with one or more sensing electrodes. These electrical properties may include electrical conductivity at one, several or a range of frequencies (e.g., in the range of from 1 kHz to 100 MHz), dielectric constant, capacitance, or combinations of these. In this embodiment, an audible signal may be produced when the sensing electrode(s) at the tip of the probe detects the fatty material surrounding a nerve, or direct feedback control can be provided to supply power to the electrode terminal(s), either individually or to the complete array of electrodes, only if and when the tissue encountered at the working end of the probe is normal tissue based on the measured electrical properties.

In one embodiment, the current limiting elements (discussed in detail above) are configured such that individual electrode terminals in an electrode array will shut down or turn off when the electrical impedance reaches a threshold level. When this threshold level is set to the impedance of the fatty material surrounding peripheral nerves, the electrode terminals will shut off whenever they come in contact with, or in close proximity to, nerves. Meanwhile, other electrode terminals, which are in contact with or in close proximity to normal target tissue, will continue to conduct electric current to the return electrode. This selective ablation or removal of lower impedance tissue, in combination with the Coblation^{™} mechanism of the present invention, allows the surgeon to precisely remove tissue around nerves or bone. Applicant has found that the present invention is capable of volumetrically removing tissue closely adjacent to nerves without impairing the function of the nerves, and without significantly damaging the tissue of the epineurium. In the present invention, the Coblation^{®} process for removing tissue results in extremely small depths of collateral tissue damage as discussed above. This allows the surgeon to remove tissue close to a nerve without causing collateral damage to the nerve fibers.

In addition to the above, applicant has discovered that the Coblation^{®} mechanism of the present invention can be manipulated to ablate or remove certain tissue structures, while having little effect on other tissue structures. As discussed above, the present invention uses a technique of vaporizing electrically conductive fluid to form a plasma layer or pocket around the electrode terminal(s), and then inducing the discharge of energy from this plasma or vapor layer to break the molecular bonds of the tissue structure. Based on initial experiments, applicants believe that the free electrons within the ionized vapor layer are accelerated in the high electric fields near the electrode tip(s). When the density of the vapor layer (or within a bubble formed in the electrically conductive liquid) becomes sufficiently low (i.e., less than approximately 10²⁰ atoms/cm³ for aqueous solutions), the electron mean free path increases to enable subsequently injected electrons to cause impact ionization within these regions of low density (i.e., vapor layers or bubbles). Energy evolved by the energetic electrons (e.g., 4 to 5 eV) can subsequently bombard a molecule and break its bonds, dissociating a molecule into free radicals, which then combine into final gaseous or liquid species.

The energy evolved by the energetic electrons may be varied by adjusting a variety of factors, such as: the number of electrode terminals; electrode size and spacing; electrode surface area; asperities and sharp edges on the electrode surfaces; electrode materials; applied voltage and power; current limiting means, such as inductors; electrical conductivity of the fluid in contact with the electrodes; density of the fluid; and other factors. Accordingly, these factors can be manipulated to control the energy level of the excited electrons. Since different tissue structures have different molecular bonds, the present invention can be configured to break the molecular bonds of certain tissue, while having too low an energy to break the molecular bonds of other tissue. For example, fatty tissue, (e.g., adipose tissue) has double bonds that require a substantially higher energy level than 4 to 5 eV to break. Accordingly, the present invention in its current configuration generally does not ablate or remove such fatty tissue. However, the present invention may be effectively used to release the inner fat content in a liquid form. Of course, factors may be changed such that these double bonds can also be broken in a similar fashion as the single bonds (e.g., increasing the voltage, or changing the electrode configuration to increase the current density at the electrode tips). A more complete description of this phenomenon can be found in co-pending U.S. Patent Application 09/032,375, filed February 27, 1998 (Attorney Docket No. CB-3).

The present invention also provides apparatus for selectively removing tumors, e.g., brain tumors, or other undesirable body structures while minimizing the spread of viable cells from the tumor. Conventional techniques for removing such tumors generally result in the production of smoke in the surgical setting, termed an electrosurgical or laser plume, which can spread intact, viable bacteria, cells, or viral particles from the tumor or lesion to the surgical team or to other portions of the patient's body. This potential spread of viable bacteria, cells, or particles has resulted in increased concerns over the proliferation of certain debilitating and fatal diseases, such as hepatitis, herpes, HIV and papillomavirus. High frequency voltage is applied between the electrode terminal(s) and one or more return electrode(s) to volumetrically remove at least a portion of the tumor tissue via the molecular dissociation of tissue components into non-viable atoms and molecules. Specifically, the present invention converts the solid tissue into non-condensable gases that are no longer intact or viable, and thus, incapable of spreading viable tumor cells or infectious particles to other parts of the patient's body or to the surgical staff. The high frequency voltage is preferably selected to effect controlled removal of these tissue cells while minimizing or avoiding substantial tissue necrosis to surrounding or underlying tissue. A more complete description of this phenomenon can be found in co-pending U.S. Patent Application 09/109,219, filed June 30, 1998 (Attorney Docket No. CB-1).

In one embodiment, an electrosurgical instrument comprises a shaft having a proximal end and a distal end which supports one or more electrode terminal(s). The shaft may assume a wide variety of configurations, with the primary purpose being to mechanically support one or more electrode terminal(s) and permit the treating physician to manipulate the electrode(s) from a proximal end of the shaft. Usually, an electrosurgical probe shaft will be a narrow-diameter rod or tube, more usually having dimensions which permit it to be introduced through a cannula into the patient's body. Thus, the probe shaft will typically have a length of at least 5 cm for open procedures and at least 10 cm, more typically being 20 cm, or longer for endoscopic procedures. The probe shaft will typically have a diameter of at least 1 mm, and frequently in the range from 1 mm to 10 mm. For dermatology or other procedures on the skin surface, the shaft will have any suitable length and diameter that would facilitate handling by the surgeon.

The electrosurgical instrument may also be a catheter that is delivered percutaneously and/or endoluminally into the patient by insertion through a conventional or specialized guide catheter, or the invention may include a catheter having an active electrode or electrode array integral with its distal end. The instrument shaft may be rigid or flexible, with flexible shafts optionally being combined with a generally rigid external tube for mechanical support. Flexible shafts may be combined with pull wires, shape memory actuators, and other known mechanisms for effecting selective deflection of the distal end of the shaft to facilitate positioning of the electrode or electrode array. The instrument shaft will usually include a plurality of wires or other conductive elements running axially therethrough to permit connection of the electrode or electrode array and the return electrode to a connection block at the proximal end of the shaft. In one embodiment, the shaft may include a rigid multi-lumen tube, wherein a return electrode lead or filament occupies a first lumen, and an active electrode lead or filament occupies a second lumen. In another embodiment, the shaft may include a guide wire for guiding the catheter to the target site, or the instrument may comprise a steerable guide catheter. A catheter may also include a substantially rigid distal end portion to increase the torque control of the distal end portion as the catheter is advanced further into the patient's body. Specific shaft designs will be described in detail in connection with the figures hereinafter.

The electrode terminal(s) may be supported by, or partially surrounded by an electrically insulating electrode support or spacer positioned at or near the distal end of the instrument shaft. The return electrode may be located at the shaft distal end, on a separate instrument, or on the external surface of the patient (i.e., a dispersive pad). In most applications, applicant has found that it is preferably to have the return electrode on or near the shaft of the instrument to confine the electric currents to the target site. In some applications and under certain conditions, however, the invention may be practiced in a monopolar mode, with the return electrode attached to the external surface of the patient. Accordingly, the return electrode is preferably either integrated with the instrument shaft, or integral with a separate instrument located in close proximity to the distal end of the instrument shaft. The proximal end of the instrument will include the appropriate electrical connections for coupling the return electrode(s) and the electrode terminal(s) to a high frequency power supply, such as an electrosurgical generator or power supply. In one embodiment, the return electrode comprises a distal portion in the form of a coil, and a proximal filament arranged within the shaft and coupled to a connection block housed within a handle of the instrument.

The current flow path between the electrode terminals and the return electrode(s) may be generated by submerging the tissue site in an electrically conductive fluid (e.g., within a viscous fluid, such as an electrically conductive gel), or by directing an electrically conductive fluid along a fluid path to the target site (i.e., a liquid, such as isotonic saline, hypotonic saline; or a gas, such as argon). The conductive gel may also be delivered to the target site to achieve a slower more controlled delivery rate of electrically conductive fluid. In addition, the viscous nature of the gel may allow the surgeon to more easily contain the gel around the target site (e.g., rather than attempting to contain isotonic saline). A more complete description of an exemplary method of directing electrically conductive fluid between the active and return electrodes is described in U.S. Patent No. 5,697,281, Alternatively, the body's natural conductive fluids, such as blood, may be sufficient to establish a conductive path between the return electrode(s) and the electrode terminal(s), and to provide the conditions for establishing a vapor layer, as described above. However, conductive fluid that is introduced into the patient is generally preferred over blood because blood will tend to coagulate at certain temperatures. In addition, the patient's blood may not have sufficient electrical conductivity to adequately form a plasma in some applications. Advantageously, a liquid electrically conductive fluid (e.g., isotonic saline) may be used to concurrently "bathe" the target tissue surface to provide an additional means for removing any tissue, and to cool the region of the target tissue ablated in the previous moment.

The power supply or electrosurgical generator may include a fluid interlock for interrupting power to the electrode terminal(s) when there is insufficient conductive fluid around the electrode terminal(s). This ensures that the instrument will not be activated when conductive fluid is not present, minimizing the tissue damage that may otherwise occur. A more complete description of such a fluid interlock can be found in commonly assigned U.S. Patent Application No. 09/058,336, filed April 10, 1998 (attorney Docket No. CB-4).

In some procedures, it may also be necessary to retrieve or aspirate the electrically conductive fluid and/or the non-condensable gaseous products of ablation. In addition, it may be desirable to aspirate small pieces of tissue or other body structures that are not completely disintegrated by the high frequency energy, or other fluids at the target site, such as blood, mucus, the gaseous products of ablation, etc. Accordingly, the system of the present invention may include one or more suction lumen(s) in the instrument, or on a separate instrument, coupled to a suitable vacuum source for aspirating excess fluids or unwanted materials from the target site. In addition, the invention may include one or more aspiration electrode(s) for ablating, or at least reducing the volume of, non-ablated tissue fragments that are aspirated into the lumen. The aspiration electrode(s) function mainly to inhibit clogging of the lumen that may otherwise occur as larger tissue fragments are drawn therein. The aspiration electrode(s) may be different from the ablation electrode terminal(s), or the same electrode(s) may serve both functions. A more complete description of instruments incorporating aspiration electrode(s) can be found in U.S. Patent Application No. 09/010,382 filed January 21, 1998.

As an alternative or in addition to suction, it may be desirable to contain the excess electrically conductive fluid, tissue fragments and/or gaseous products of ablation at or near the target site with a containment apparatus, such as a basket, retractable sheath or the like. This embodiment has the advantage of ensuring that the conductive fluid, tissue fragments or ablation products do not flow through the patient's vasculature or into other portions of the body. In addition, it may be desirable to limit the amount of suction to limit the undesirable effect suction may have on hemostasis of severed blood vessels.

The present invention may use a single active electrode terminal or an array of electrode terminals spaced around the distal surface of a catheter or probe. In the latter embodiment, the electrode array usually includes a plurality of independently current-limited and/or power-controlled electrode terminals to apply electrical energy selectively to the target tissue while limiting the unwanted application of electrical energy to the surrounding tissue resulting from power dissipation into surrounding electrically conductive fluids, such as blood, normal saline, and the like. The electrode terminals may be independently current-limited by isolating the terminals from each other and connecting each terminal to a separate power source that is isolated from the other electrode terminals. Alternatively, the electrode terminals may be connected to each other at either the proximal or distal ends of the catheter to form a single wire that couples to a power source.

In one configuration, each individual electrode terminal in the electrode array is electrically insulated from all other electrode terminals in the array within said instrument and is connected to a power source which is isolated from each of the other electrode terminals in the array or to circuitry which limits or interrupts current flow to the electrode terminal when low resistivity material (e.g., blood, electrically conductive saline irrigant or electrically conductive gel) causes a lower impedance path between the return electrode and the individual electrode terminal. The isolated power sources for each individual electrode terminal may be separate power supply circuits having internal impedance characteristics which limit power to the associated electrode terminal when a low impedance return path is encountered. By way of example, the isolated power source may be a user selectable constant current source. In this embodiment, lower impedance paths will automatically result in lower resistive heating levels since the heating is proportional to the square of the operating current times the impedance. Alternatively, a single power source may be connected to each of the electrode terminals through independently actuatable switches, or by independent current limiting elements, such as inductors, capacitors, resistors and/or combinations thereof. The current limiting elements may be provided in the instrument, connectors, cable, controller, or along the conductive path from the controller to the distal tip of the instrument. Alternatively, the resistance and/or capacitance may occur on the surface of the active electrode terminal(s) due to oxide layers which form selected electrode terminals (e.g., titanium or a resistive coating on the surface of metal, such as platinum).

The tip region of the instrument may comprise many independent electrode terminals designed to deliver electrical energy in the vicinity of the tip. The selective application of electrical energy to the conductive fluid is achieved by connecting each individual electrode terminal and the return electrode to a power source having independently controlled or current limited channels. The application of high frequency voltage between the return electrode and the electrode array results in the generation of high electric field intensities at the distal tips of the electrode terminals with conduction of high frequency current from each individual electrode terminal to the return electrode. The current flow from each individual electrode terminal to the return electrode is controlled by either active or passive means, or a combination thereof, to deliver electrical energy to the surrounding conductive fluid while minimizing energy delivery to surrounding (non-target) tissue.

The application of a high frequency voltage between the return electrode(s) and the electrode terminal(s) for appropriate time intervals effects cutting, removing, ablating, shaping, contracting, or otherwise modifying the target tissue. The tissue volume over which energy is dissipated (i.e., over which a high current density exists) may be more precisely controlled, for example, by the use of a multiplicity of small electrode terminals whose effective diameters or principal dimensions range from about 10 mm to 0.01 mm, preferably from about 2 mm to 0.05 mm, and more preferably from about 1 mm to 0.1 mm. Electrode areas for both circular and non-circular terminals will have a contact area (per electrode terminal) below 50 mm² for electrode arrays and as large as 75 mm² for single electrode embodiments. In multiple electrode arrays, the contact area of each electrode terminal is typically in the range from 0.0001 mm² to 1.0 mm², and more preferably from 0.001 mm² to 0.5 mm². The circumscribed area of the electrode array or electrode terminal is in the range from 0.25 mm² to 75 mm², preferably from 0.5 mm² to 40 mm².

The distal or working end of the instrument, including the active and return electrodes, can assume a variety of geometries, with particular geometries and configurations being selected for specific applications. Typically, the active electrode(s) or electrode terminal(s) are located at the distal tip of the electrosurgical instrument. Alternatively or additionally, the active electrode(s) may be formed on lateral surfaces of the electrosurgical instrument shaft, e.g., for facilitating access to certain body structures during endoscopic procedures.

The electrically conductive fluid should have an electrical conductivity above a minimum threshold level to provide a suitable current flow path between the return electrode and the electrode terminal(s). The electrical conductivity of the fluid (in units of milliSiemens per centimeter or mS/cm) will usually be greater than 0.2 mS/cm, preferably will be greater than 2 mS/cm and more preferably greater than 10 mS/cm. In an exemplary embodiment, the electrically conductive fluid is isotonic saline, which has a conductivity of about 17 mS/cm. Applicant has found that a more conductive fluid, or one with a higher ionic concentration, will usually provide a more aggressive ablation rate. For example, a saline solution with higher levels of sodium chloride than conventional saline (which is on the order of about 0.9% sodium chloride) e.g., on the order of greater than 1% or between about 3% and 20%, may be desirable. Alternatively, the invention may be used with different types of conductive fluids that increase the power of the plasma layer by, for example, increasing the quantity of ions in the plasma, or by providing ions that have higher energy levels than sodium ions. For example, the present invention may be used with elements other than sodium, such as potassium, magnesium, calcium, and the like. In addition, other electronegative elements may be used in place of chlorine, such as fluorine.

The voltage difference applied between the return electrode(s) and the electrode terminal(s) will be at high or radio frequency (RF), typically between about 5 kHz and 20 MHz, usually being between about 30 kHz and 2.5 MHz, preferably being between about 50 kHz and 500 kHz, often less than 350 kHz, and often between about 100 kHz and 200 kHz. In some applications, applicant has found that a frequency of about 100 kHz is useful because the tissue impedance is much greater at this frequency. In other applications, such as procedures in or around the heart or head and neck, higher frequencies may be desirable (e.g., 400 to 600 kHz) in order to minimize low frequency current flow into the heart or the nerves of the head and neck. The RMS (root mean square) voltage applied will usually be in the range of from about 5 volts RMS to 1000 volts RMS, typically being in the range from about 10 volts RMS to 500 volts RMS, and often between about 150 volts RMS to 350 volts RMS, depending on the electrode terminal size, the operating frequency, and the operation mode of the particular procedure, or desired effect on the tissue (e.g., contraction, coagulation, cutting or ablation). Typically, the peak-to-peak voltage for ablation or cutting with a square wave form will be in the range of from about 10 to 2000 volts, preferably in the range of 100 to 1800 volts, and more preferably in the range of about 300 to 1500 volts, often in the range of about 300 to 800 volts peak to peak (again, depending on the electrode size, the operating frequency, and the operation mode). Lower peak-to-peak voltages may be used for tissue coagulation or collagen contraction, and will typically be in the range of from about 50 to 1500, preferably 100 to 1000, and more preferably 120 to 400 volts peak-to-peak (again, these values are computed using a square wave form). Higher peak-to-peak voltages, e.g., greater than about 700 volts peak-to-peak, may be desirable for ablation of harder material, such as bone, depending on other factors, such as the electrode geometries and the composition of the conductive fluid.

As discussed above, the voltage is usually delivered in a series of voltage pulses or alternating current of time varying voltage amplitude with a sufficiently high frequency (e.g., on the order of 5 kHz to 20 MHz) such that the voltage is effectively applied continuously (as compared with e.g., lasers claiming small depths of necrosis, which are generally pulsed at about 10 to 20 Hz). In addition, the duty cycle (i.e., cumulative time in any one-second interval that energy is applied) is on the order of about 50% for the present invention, as compared with pulsed lasers which typically have a duty cycle of about 0.0001 %.

The preferred power source of the present invention delivers a high frequency current selectable to generate average power levels ranging from several milliwatts to tens of watts per electrode, depending on the volume of target tissue being treated, and/or the maximum allowed temperature selected for the instrument tip. The power source allows the user to select the voltage level according to the specific requirements of a particular procedure, e.g., neurosurgery, cardiac surgery, arthroscopic surgery, dermatological procedure, ophthalmic procedures, open surgery, or various endoscopic surgical procedures. For cardiac procedures, and potentially for neurosurgery, the power source may have an additional filter, for filtering leakage voltages at frequencies below about 100 kHz, particularly voltages around 60 kHz. Alternatively, a power source having a higher operating frequency, e.g., from about 300 to 600 kHz may be used in certain procedures in which stray low frequency currents may be problematic. A description of one suitable power source can be found in co-pending Patent Applications 091058,571 and 09/058,336, filed April 10, 1998 (Attorney Docket Nos. CB-2 and CB-4).

The power source may be current limited or otherwise controlled so that undesired heating of the target tissue or surrounding (non-target) tissue does not occur. In a presently preferred embodiment of the present invention, current limiting inductors are placed in series with each independent electrode terminal, where the inductance of the inductor is in the range of 10µH to 50,000µH, depending on the electrical properties of the target tissue, the desired tissue heating rate, and the operating frequency. Alternatively, capacitor-inductor (LC) circuit structures may be employed, as described previously in U.S. Patent No. 5,697,909, the complete disclosure of which is incorporated herein by reference. Additionally, current-limiting resistors may be selected. Preferably, these resistors will have a large positive temperature coefficient of resistance so that, as the current level begins to rise for any individual electrode terminal in contact with a low resistance medium (e.g., saline irrigant or blood), the resistance of the current limiting resistor increases significantly, thereby minimizing the power delivery from said electrode terminal into the low resistance medium (e.g., saline irrigant or blood).

In one embodiment, the instrument incorporates a single active electrode that extends directly through the shaft and is connected by a single lead or filament to a connection block that is adapted for facile coupling to a high frequency power supply. The active electrode(s) may have ball shapes (e.g., for tissue vaporization and desiccation), twizzle shapes (for vaporization and needle-like cutting), spring shapes (for rapid tissue debulking and desiccation), twisted metal shapes, annular or solid tube shapes or the like. Alternatively, the electrode(s) may comprise a plurality of filaments, rigid or flexible brush electrode(s) (for debulking a tumor, such as a fibroid, bladder tumor or a prostate adenoma), side-effect brush electrode(s) on a lateral surface of the shaft, coiled electrode(s) or the like. In an exemplary embodiment, the active electrode comprises a flattened electrode head, e.g., in the form of a flattened 1-turn coil, or a disc.

In one embodiment, an electrosurgical catheter or probe comprises a single active electrode terminal encircled along a portion of its length by an electrically insulating spacer, e.g., comprising a ceramic. The insulating member may be a tubular or cylindrical structure that separates the active electrode terminal from a distal portion of the return electrode, the return electrode lying external to the insulating spacer.

The current flow path between the electrode terminal(s) and the return electrode(s) may be generated by submerging the tissue site in an electrically conductive fluid (e.g., within a viscous fluid, such as an electrically conductive gel) or by directing an electrically conductive fluid along a fluid path to the return electrode and/or the active electrode. This latter method is particularly effective in a dry environment (i.e., the tissue is not submerged in fluid) because the electrically conductive fluid provides a suitable current flow path from the active electrode head or terminal to the return electrode.

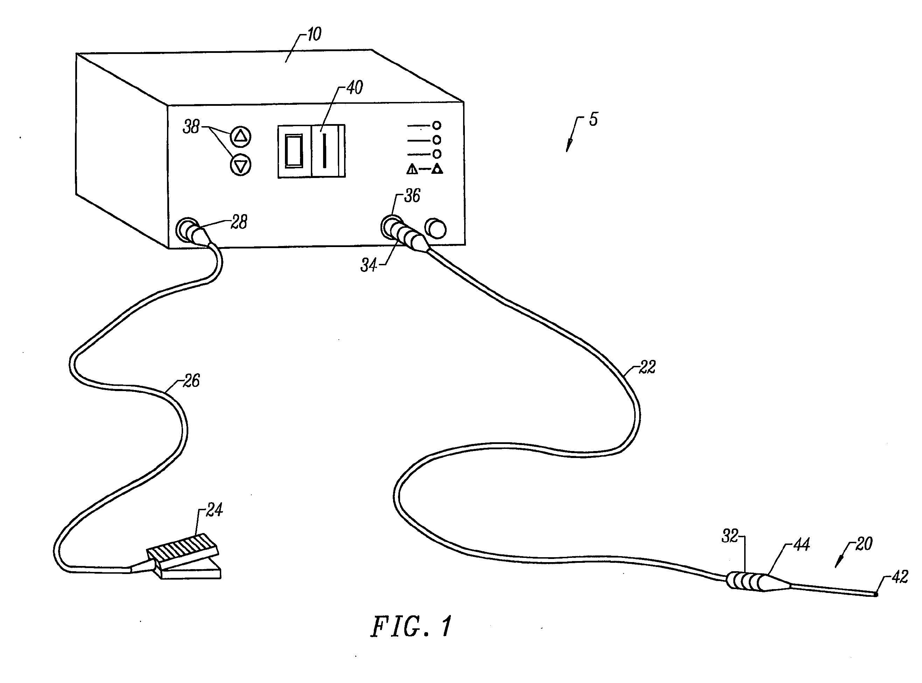

Referring to Fig. 1, an exemplary electrosurgical system 5 for treatment of tissue in the body will now be described in detail. As shown, electrosurgical system 5 generally includes an electrosurgical probe 20 connected to a power supply 10 for providing high frequency voltage to one or more electrode terminals 42 on probe 20. Probe 20 includes a connector housing 44 at its proximal end, which can be removably connected to a probe receptacle 32 of a probe cable 22. The proximal portion of cable 22 has a connector 34 to couple probe 20 to power supply 10. Power supply 10 has an operator controllable voltage level adjustment 38 to change the applied voltage level, which is observable at a voltage level display 40. Power supply 10 also includes one or more foot pedal(s) 24 and one or more cable(s) 26 which are each removably coupled to receptacle 30 with a cable connector 28. The foot pedal(s) 24 may include a second pedal (not shown) for remotely adjusting the energy level applied to electrode terminals 42, and a third pedal (also not shown) for switching between an ablation mode and a sub-ablation mode (e.g., for coagulation or contraction of tissue).

In one embodiment, a first foot pedal is used to place the power supply into the ablation mode and a second foot pedal (not shown) places power supply 10 into the sub-ablation mode. A third foot pedal (not shown) allows the user to adjust the voltage level within the ablation mode. In the ablation mode, a sufficient voltage is applied to the electrode terminals to establish the requisite conditions for molecular dissociation of the tissue. As discussed above, the requisite voltage level for ablation will vary depending on the number, size, shape and spacing of the electrodes, and the distance to which the electrodes extend from the probe distal end. When the surgeon is using the power supply in the ablation mode, voltage level adjustment 38 or the third foot pedal may be used to adjust the voltage level to adjust the degree or aggressiveness of the ablation. Of course, it will be recognized that the voltage and modality of the power supply may also be controlled by other input devices.

In the sub-ablation mode, power supply 10 applies a low enough voltage to one or more electrode terminals (or one or more coagulation electrodes) to avoid vaporization of the electrically conductive fluid, formation of a plasma, and subsequent molecular dissociation of the tissue. The surgeon may automatically toggle the power supply between the ablation and sub-ablation modes by alternatively stepping on the appropriate foot pedals. This allows the surgeon to quickly move between coagulation and ablation *in situ,* without having to remove his/her concentration from the surgical field, or without having to request an assistant to switch the power supply. By way of example, as the surgeon is treating soft tissue in the ablation mode, the probe typically will simultaneously seal and/or coagulate small severed vessels within the tissue. However, larger vessels, or vessels with high fluid pressures (e.g., arterial vessels) may not be sealed in the ablation mode. Accordingly, the surgeon can simply actuate the appropriate foot pedal, automatically lowering the voltage level below the threshold level for ablation, and apply sufficient pressure onto the severed vessel for a sufficient period of time to seal and/or coagulate the vessel. After hemostasis is achieved, the surgeon may quickly move back into the ablation mode by actuating the appropriate foot pedal. A specific design of a suitable power supply for use with the present invention can be found in U.S. Patent Application No. 09/058,571, filed April 10, 1998 (attorney docket no. CB-2).

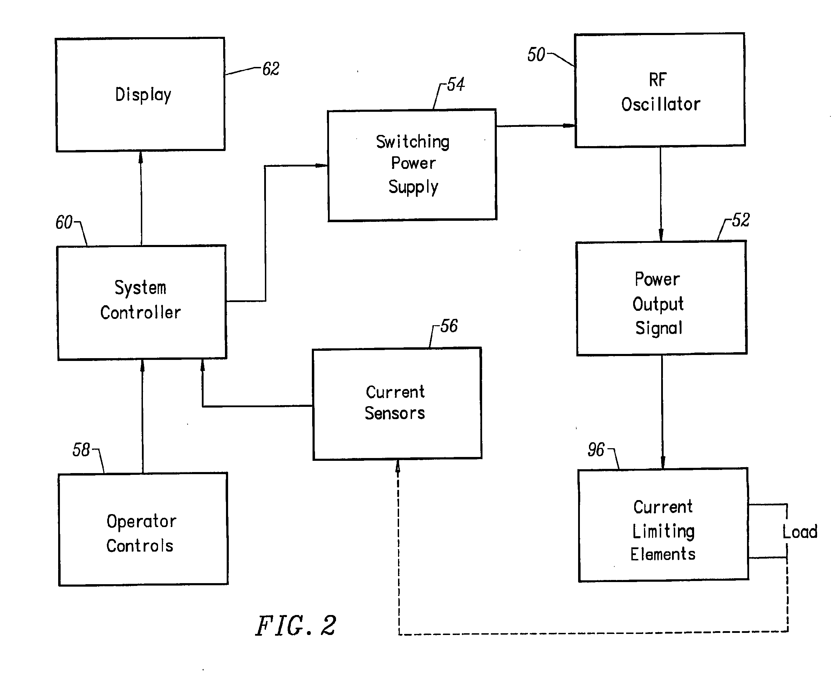

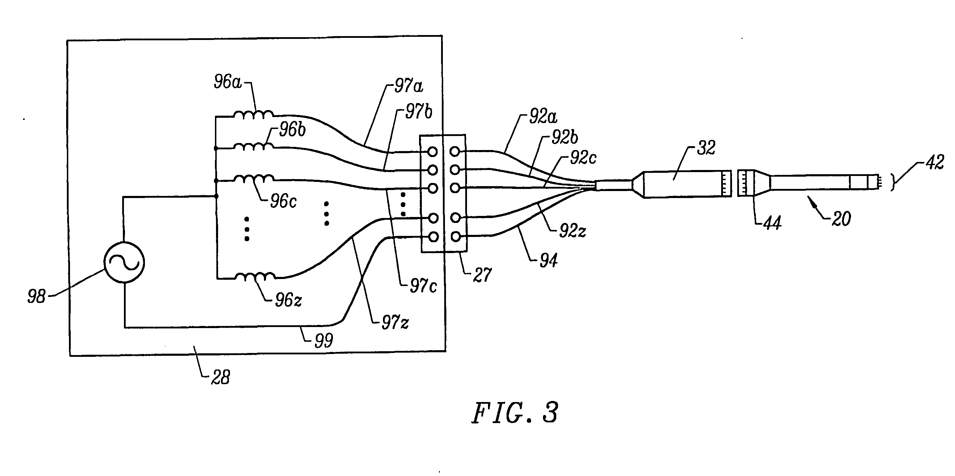

Referring now to Figs. 2 and 3, a representative high frequency power supply or generator for use according to the principles of the present invention will now be described. The high frequency power supply of the present invention is configured to apply a high frequency voltage of from about 10 volts RMS to 500 volts RMS between one or more electrode terminals (and/or coagulation electrode) and one or more return electrodes. In the exemplary embodiment, the power supply applies from about 70 volts RMS to 500 volts RMS in the ablation mode, and from about 10 volts RMS to 90 volts RMS in the sub-ablation mode, preferably from about 45 to 70 volts RMS in the sub-ablation mode (these values will, of course, vary depending on the probe configuration attached to the power supply and the desired mode of operation).

The preferred power source of the present invention delivers a high frequency current selectable to generate average power levels ranging from several milliwatts to tens of watts per electrode, depending on the volume of target tissue being treated, and/or the maximum allowed temperature selected for the probe tip. The power source allows the user to select the voltage level according to the specific requirements of a particular procedure, e.g., arthroscopic surgery, dermatological procedure, ophthalmic procedures, open surgery, or other endoscopic surgery procedure.

As shown in Fig. 2, the power supply or generator generally comprises a radio frequency (RF) power oscillator 50 having output connections for coupling via a power output signal 52 to the load impedance, which is represented by the electrode assembly when the electrosurgical probe is in use. In the representative embodiment, the RF oscillator operates at about 100 kHz. The RF oscillator is not limited to this frequency and may operate at frequencies of from about 300kHz to 600kHz. In particular, for cardiac applications, the RF oscillator will preferably operate in the range of from about 400 kHz to 600 kHz. The RF oscillator will generally supply a square wave signal with a crest factor of about 1 to 2. Of course, this signal may be a sine wave signal or other suitable wave signal depending on the application and other factors, such as the voltage applied, the number and geometry of the electrodes, etc. The power output signal 52 is designed to incur minimal voltage decrease (i.e., sag) under load. This improves the applied voltage to the electrode terminals and the return electrode, which improves the rate of volumetric removal (ablation) of tissue.

Power is supplied to the oscillator 50 by a switching power supply 54 coupled between the power line and RF oscillator 50 rather than a conventional transformer. The switching power supply 54 allows the generator to achieve high peak power output without the large size and weight of a bulky transformer. The architecture of switching power supply 54 has also been designed to reduce electromagnetic noise such that U.S. and foreign EMI requirements are met. This architecture comprises a zero voltage switching or crossing, which causes the transistors to turn ON and OFF when the voltage is zero. Therefore, the electromagnetic noise produced by the transistors switching is vastly reduced. In an exemplary embodiment, the switching power supply 54 operates at about 100 kHz.

A system controller 60, coupled to the operator controls 58 (e.g., foot pedals and hand-actuated voltage selector) and a display 62, is connected to a control input of switching power supply 54 for adjusting the generator output power by supply voltage variation. System controller 60 may be a microprocessor or an integrated circuit. The generator may also include one or more current sensors 56 for detecting the output current. The generator is preferably housed within a metal casing which provides a durable enclosure for the electrical components therein. In addition, the metal casing reduces the electromagnetic noise generated within the power supply because the grounded metal casing functions as a "Faraday shield", thereby shielding the environment from internal sources of electromagnetic noise.

The generator generally comprises a main or mother board containing generic electrical components required for many different surgical procedures (e.g., arthroscopy, urology, general surgery, dermatology, neurosurgery, etc.), and a daughter board containing application specific current-limiting circuitry (e.g., inductors, resistors, capacitors, and the like). The daughter board is coupled to the mother board by a detachable multi-pin connector to allow convenient conversion of the power supply to, e.g., applications requiring a different current limiting circuit design. For arthroscopy, for example, the daughter board preferably comprises a plurality of inductors of about 200 to 400 µH, usually about 300 µH, for each of the channels supplying current to the electrode terminals (see Fig. 3).

Alternatively, in one embodiment, current limiting inductors are placed in series with each independent electrode terminal, where the inductance of the inductor is in the range of 10µH to 50,000µH, depending on the electrical properties of the target tissue, the desired tissue heating rate, and the operating frequency. Alternatively, capacitor-inductor (LC) circuit structures may be employed, as described previously in PCT application No. PCT/US94/05168. Additionally, current limiting resistors may be selected. Preferably, these resistors will have a large positive temperature coefficient of resistance so that, as the current level begins to rise for any individual electrode terminal in contact with a low resistance medium (e.g., saline irrigant or conductive gel), the resistance of the current limiting resistor increases significantly,thereby minimizing the power delivery from the electrode terminal into the low resistance medium (e.g., saline irrigant or conductive gel). Power output signal 52 may also be coupled to a plurality of current limiting elements 96, which are preferably located on the daughter board since the current limiting elements may vary depending on the application.

Fig. 3 illustrates an arrangement that may be used in various electrosurgical procedures with a multi-electrode probe. As shown, a high frequency power supply 28 comprises a voltage source 98 which is connected to a multiplicity of current limiting elements 96a, 96b, ... 96z, typically being inductors having an inductance in the range of about 100 to 5000 µH, with the particular value depending on the electrode terminal dimensions, the desired ablation rates, and the like. Capacitors having capacitance values in the range of about 200 to 10,000 picofarads may also be used as the current limiting elements. It would also be possible to use resistors as current limiting elements. The current limiting elements may also be part of a resonant circuit structure, as described in detail in PCT Application No. PCT/US94/05168.

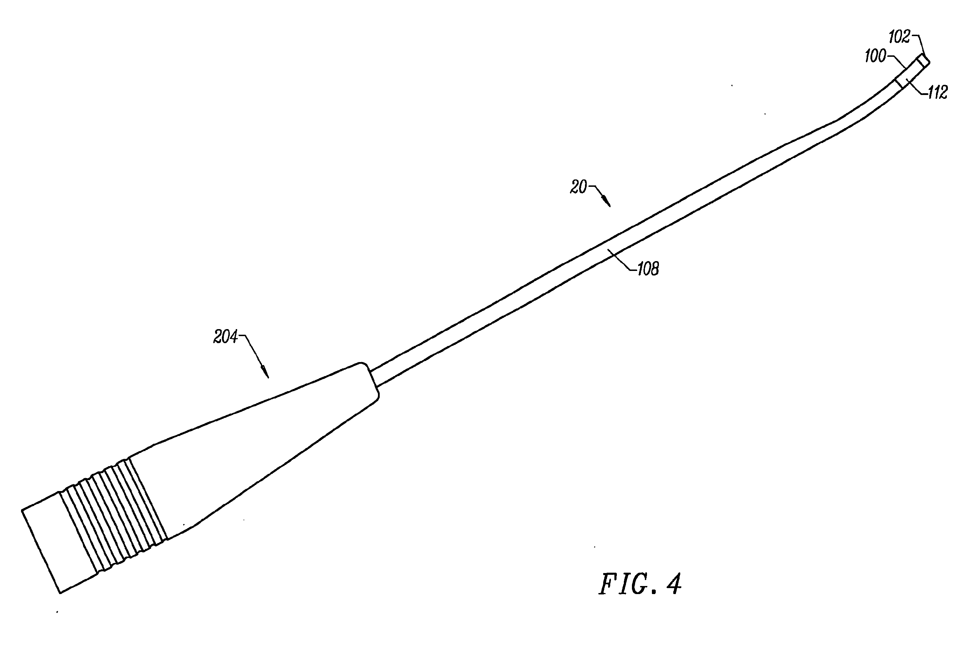

Fig. 4 illustrates a probe 20, which generally includes an elongated shaft 100, a handle 204 coupled to the proximal end of shaft 100, and an electrode support member or spacer 102 coupled to the distal end of shaft 100. In the embodiment shown in Fig. 4, electrode support member 102 extends from the distal end of shaft 100 (usually by about 1 mm to 20 mm), and provides support for one or more electrically isolated electrode terminals (not shown in Fig. 4). Electrode support member 102 is typically a silicone rubber, a ceramic, a glass, or a glass/ceramic composition (e.g., aluminum oxide, titanium nitride, or the like). Alternatively, electrode support member 102 may comprise a high-temperature biocompatible plastic, such as polyether-ether-ketone (PEEK) (Vitrex International Products, Inc.), or polysulfone (GE Plastics). A return electrode 112 is located proximal to support member 102.

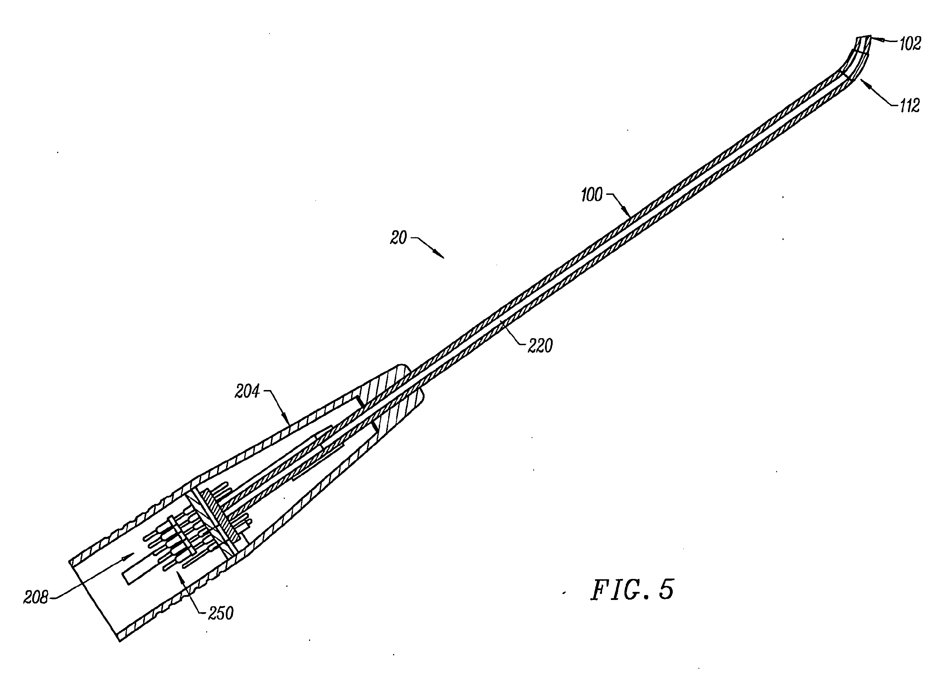

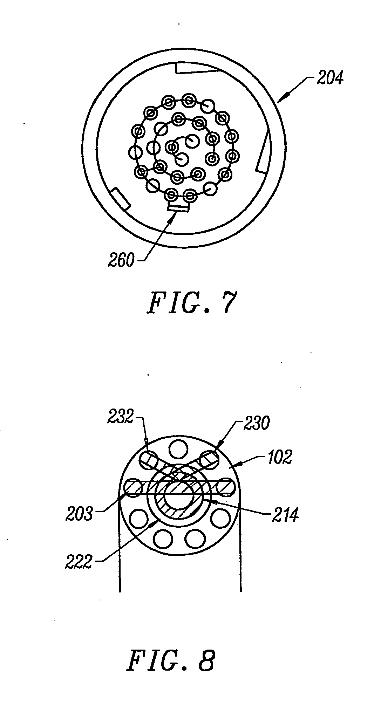

Handle 204 typically comprises a plastic material that is easily molded into a suitable shape for handling by the surgeon. As shown in Fig. 5, handle 204 defines an inner cavity 208 that houses the electrical connections 250, and provides a suitable interface for coupling probe 20 to an electrical connecting cable 22 (see Fig. 1). As shown in Fig. 7, the probe will typically include a coding resistor 260 having a value selected to program different output ranges and modes of operation for the power supply. This allows a single power supply to be used with a variety of different probes in different applications (e.g., dermatology, cardiac surgery, neurosurgery, arthroscopy, etc).

In some embodiments, probe 20 further includes an identification element that is characteristic of the particular electrode assembly so that the same power supply 28 can be used for different electrosurgical operations. In one embodiment, for example, the probe 20 includes a voltage reduction element or a voltage reduction circuit for reducing the voltage applied between the electrode terminals and return electrode 112. The voltage reduction element serves to reduce the voltage applied by the power supply so that the voltage between the electrode terminals and return electrode 112 is low enough to avoid excessive power dissipation into the electrically conductive medium, and/or to avoid excessive ablation of the tissue at the target site. The voltage reduction element primarily allows the electrosurgical probe 20 to be compatible with a range of electrosurgical generators (e.g., various generators supplied by ArthroCare Corporation, Sunnyvale, CA) that are adapted to apply higher voltages for ablation of tissue. For example, for contraction of tissue, the voltage reduction element will serve to reduce a voltage of about 100 to 135 volts RMS to about 45 to 60 volts RMS, which is a suitable voltage for contraction of tissue without ablating the tissue. Of course, for some procedures, the probe will typically not require a voltage reduction element. Alternatively, the probe may include a voltage increasing element or circuit, if desired.

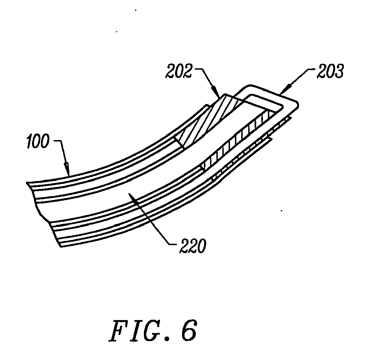

Figs. 5-8 illustrate another embodiment of the present invention, incorporating an aspiration lumen and a loop electrode designed to ablate tissue fragments as they are aspirated into the aspiration lumen. As shown in Fig. 5, electrosurgical probe 20 includes an elongated shaft 100 which may be flexible or rigid, a handle 204 coupled to the proximal end of shaft 100, an electrode support member 102 coupled to the distal end of shaft 100, and a return electrode 112 disposed proximal to support member 102. As shown in Fig. 6, probe 20 includes an active loop electrode 203. Return electrode 112(not shown in Fig. 6) is spaced proximally from active loop electrode 203. The probe 200 further includes a suction lumen 220 for aspirating excess fluids, bubbles, tissue fragments, and/or products of ablation from the target site. As shown in Figs. 5 and 6, suction lumen 220 extends through support member 102, and terminates in a distal opening 222 (Fig. 8), and extends proximally to an external connector for coupling to a vacuum source (the latter well known in the art).

Again with reference to Fig. 5, electrode support member 102 extends from the distal end of shaft 100 and provides support for loop electrode 203 and a ring electrode 214 (Fig. 8). As shown in Fig. 8, loop electrode 203 has first and second ends extending from electrode support member 102. The first and second ends are each coupled to, or integral with, one or more connectors, e.g., wires, leads, or filaments (not shown), that extend through shaft 100 to the probe proximal end for coupling to the high frequency power supply. The loop electrode 203 usually extends about 0.5 to 10 mm from the distal end of support member 102, often about 1 to 2 mm. Loop electrode 203 usually extends further away from support member 102 than ring electrode 214, in order to facilitate ablation of tissue. As discussed below, loop electrode 203 is especially configured for tissue ablation, while ring electrode 214 ablates tissue fragments that are aspirated towards distal opening 222.

Referring to Fig. 8, ring electrode 214 preferably comprises a tungsten or titanium wire having two ends 230, 232 coupled to electrical connectors (not shown) within support member 102. The wire is bent to form one-half of a figure eight, thereby forming a ring positioned over opening 222 of suction lumen 220. This ring inhibits passage of tissue fragments large enough to clog suction lumen 220. Moreover, voltage applied between ring electrode 214 and the return electrode provide sufficient energy to ablate these tissue fragments into smaller fragments that are then aspirated through lumen 220. Typically, ring electrode 214 and loop electrode 203 are electrically isolated from each other. However, these electrodes 214, 203 may be electrically coupled in some applications.

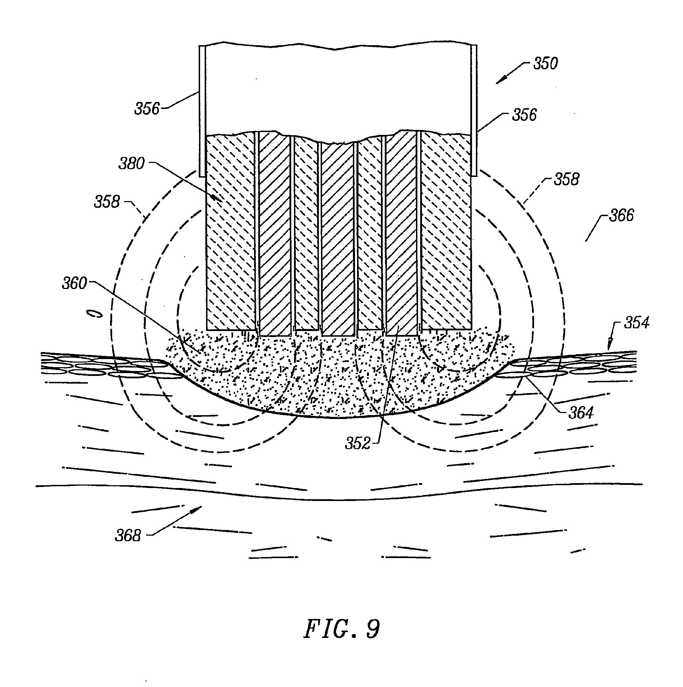

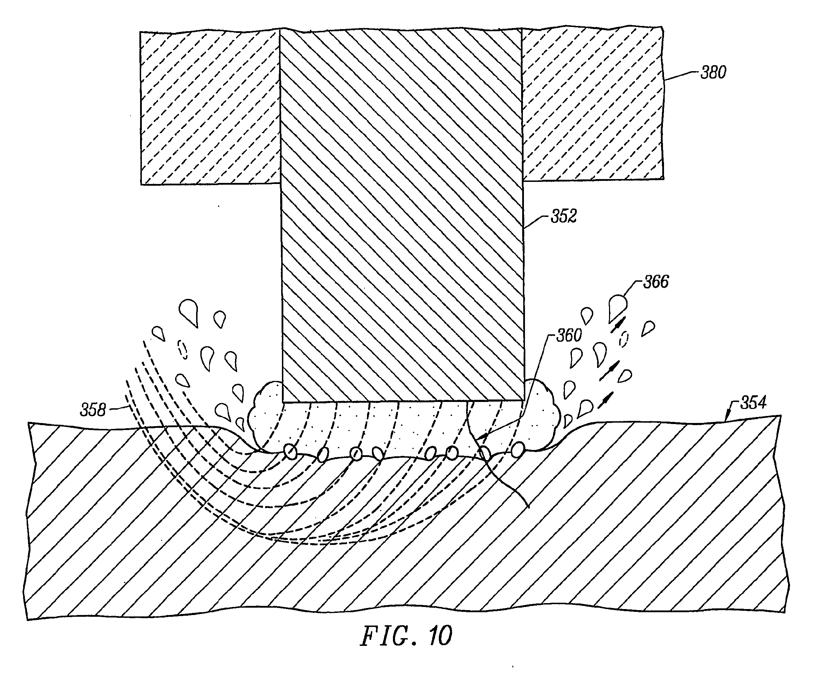

Figs. 9 and 10 illustrate use of a probe 350 of the present invention for ablating tissue. As shown, the distal portion of probe 350 is introduced to the target site (either endoscopically, through an open procedure, or directly on the patient's skin) and electrode(s) 352 are positioned adjacent to a target tissue. Fig. 9 illustrates a probe having multiple active electrodes 352. while Fig. 10 illustrates a probe having a single active electrode 352. In one embodiment, the target site is immersed in an electrically conductive fluid, such that the electrically conductive fluid generates a current flow path (see current flux lines 358) between a return electrode 356 and the active electrode(s) 352, and whereby the zone between the tissue 354 and electrode support 380 is constantly immersed in the electrically conductive fluid. The power supply (not shown) is then turned on and adjusted such that a high frequency voltage difference is applied between electrode terminal(s) 352 and return electrode 356

In the representative embodiment, the high frequency voltage is sufficient to convert the electrically conductive fluid between the target tissue 354 and active electrode terminal(s) 352 into an ionized vapor layer or plasma 360. As a result of the applied voltage difference between active electrode terminal(s) 352 and the target tissue 354 (i.e., the voltage gradient across the plasma layer 360, charged particles in the plasma (e.g., electrons) are accelerated towards the tissue. At sufficiently high voltage differences, these charged particles gain sufficient energy to cause dissociation of the molecular bonds within tissue structures. This molecular dissociation is accompanied by the volumetric removal (i.e., ablative sublimation) of tissue, and the production of low molecular weight gases 366, such as oxygen, nitrogen, carbon dioxide, hydrogen, and methane. This process can be precisely controlled, whereby damage to the underlying (non-target) tissue 368 is minimized or avoided.

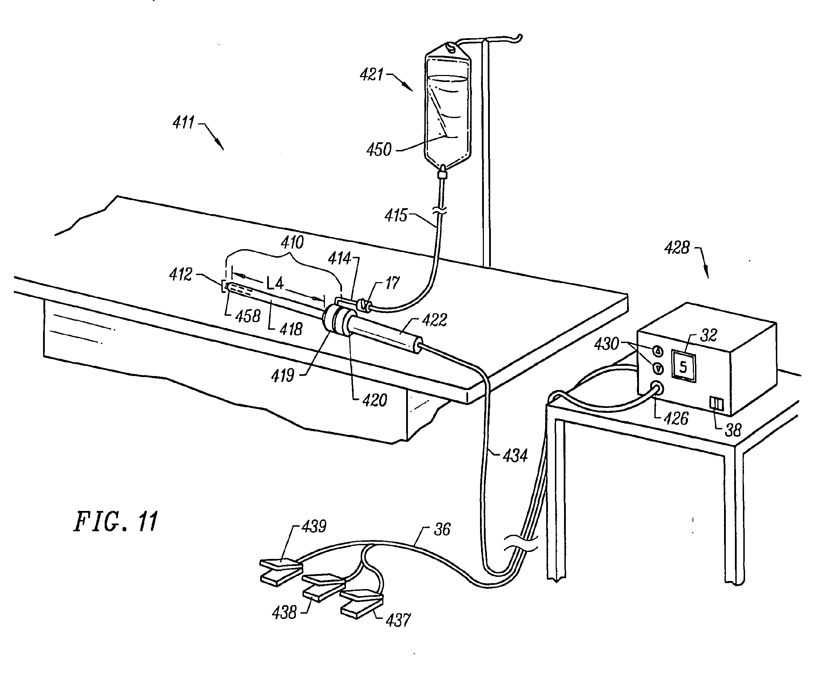

Fig. 11 schematically represents an electrosurgical system 411, according to another embodiment of the invention. System 411 is particularly useful in 'dry fields' where an electrically conductive fluid is preferably delivered via an electrosurgical probe 410 to the target site. Of course, system 411 may also be used in 'wet fields', i.e., the target site is immersed in an electrically conductive fluid. As shown, electrosurgical system 411 generally includes probe 410 connected to a power supply 428 for providing a high frequency voltage to probe 410, and a fluid source 421 for supplying electrically conductive fluid 450 to probe 410. In addition, electrosurgical system 411 may include an endoscope (not shown) with a fiber optic head light for viewing the surgical site. The endoscope may be integral with probe 410, or it may be part of a separate instrument. System 411 further includes first, second, and third foot pedals 437, 438, 439, for adjusting the voltage level of power supply 428, generally as described hereinabove. System 411 may also include an aspiration or suction element for aspirating excess fluid or unwanted materials from the surgical site.

As shown, probe 410 generally includes a proximal handle 419 and an elongate shaft 418 having an array 412 of electrode terminals 458 at its distal end. A connecting cable 434 has a connector 426 for electrically coupling the electrode terminals 458 to power supply 428. The electrode terminals 458 are electrically isolated from each other, and each of the terminals 458 is connected to an active or passive control network within power supply 428 by means of a plurality of individually insulated conductors (not shown). A fluid supply tube 415 is connected to probe 410 for supplying electrically conductive fluid 450 to the distal end of probe 410 or to the target site.

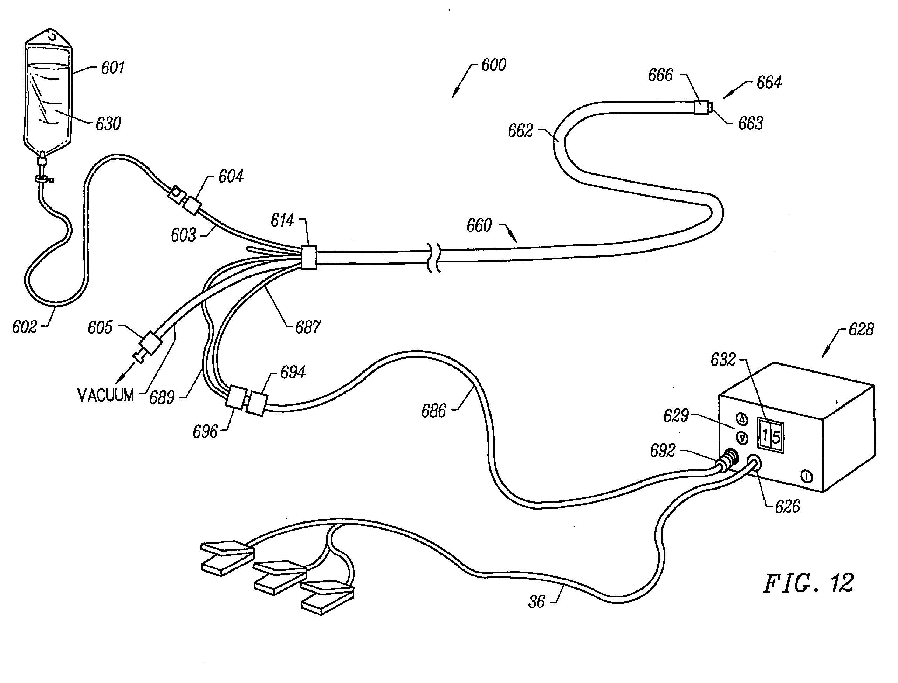

Referring to Fig. 12, an electrosurgical device according to the present invention may also be configured as an elongate catheter system 600, including portions with sufficient flexibility to permit introduction into the body and to the target site through one or more vascular lumen(s). As shown, system 600 generally comprises an electrosurgical catheter 660 connected to a power supply or generator 628 by an interconnecting cable 686 for providing high frequency voltage between active electrode(s) 663 and return electrode(s) 666, and an irrigant reservoir or fluid source 601 for providing electrically conductive fluid 630 to the target site. Catheter 660 generally comprises an elongate, flexible shaft body 662 including a tissue removing or ablating region 664 at the distal end of body 662. The proximal portion of catheter 660 includes a multi-lumen fitment 614 which provides for interconnections between lumens and electrical leads within catheter 660, and conduits and cables proximal to fitment 614. By way of example, a catheter electrical connector 696 is removably connected to a cable connector 694 which, in turn, is removably connectable to power supply 628 through connector 692. One or more electrically conducting lead wires (not shown) within catheter 660 extend between one or more active electrodes 663 at tissue ablating region 664 and one or more corresponding electrical terminals (also not shown) in catheter connector 696 via active electrode cable branch 687. Similarly, one or more return electrodes 666 at tissue ablating region 664 are coupled to a return electrode cable branch 689 of catheter connector 696 by lead wires (not shown). Of course, a single cable branch (not shown) may be used for both active and return electrodes.

Catheter body 662 may include reinforcing fibers or braids (not shown) in the walls of at least the distal ablating region 664 of body 662 to provide responsive torque control for rotation of ablating region 664 during tissue engagement. This rigid portion of the catheter body 662 typically extends only about 7 to 10 mm while the remainder of catheter body 662 is flexible to provide good trackability during advancement and positioning of active electrode(s) 663 adjacent target tissue.

Conductive fluid 630 is provided to tissue ablating region 664 of catheter 660 via a lumen (not shown in Fig. 12) within catheter 660. Fluid is supplied to the lumen from fluid source 601 along a conductive fluid supply line 602 and a conduit 603, which is coupled to the inner catheter lumen at multi-lumen fitment 614. A control valve 604 may be positioned at the interface of fluid supply line 602 and conduit 603 to allow manual control of the flow rate of electrically conductive fluid 630. Alternatively, a metering pump or flow regulator may be used to precisely control the flow rate of the conductive fluid. System 600 may further include an aspiration or vacuum system (not shown) to aspirate liquids and gases from the target site. The aspiration system will usually comprise a vacuum source coupled to fitment 614 by an aspiration connector 605.

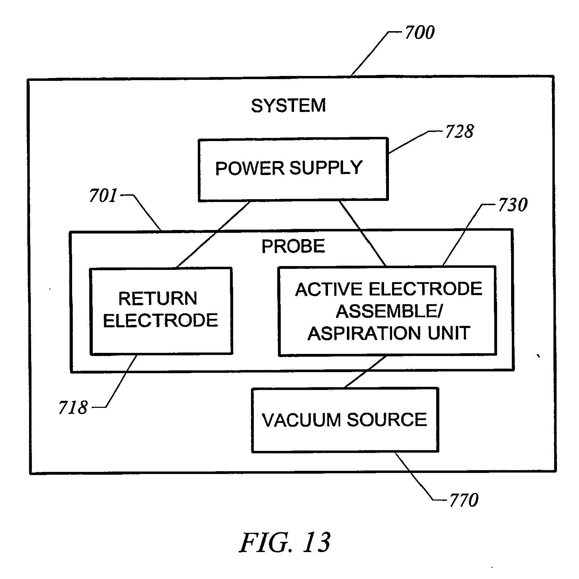

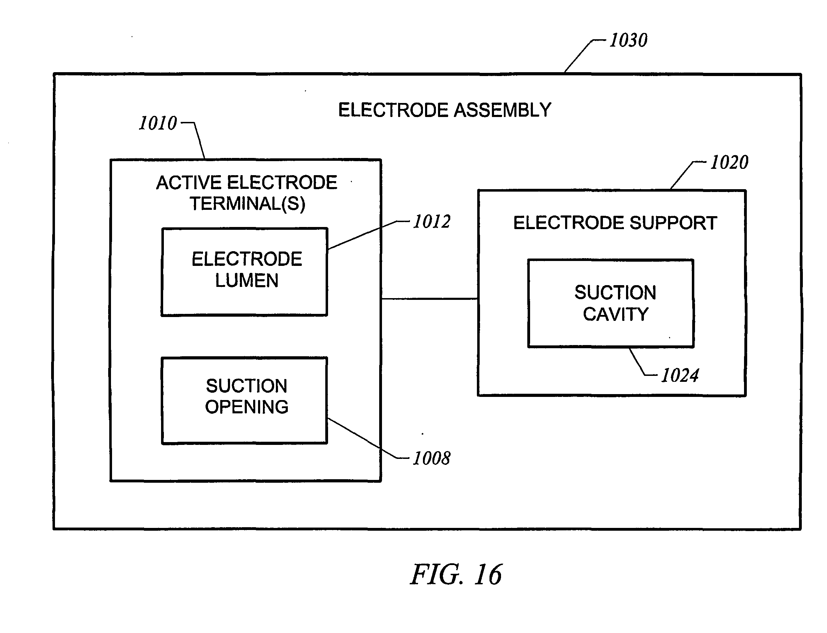

Fig. 13 is a block diagram schematically representing an electrosurgical system 700, according to another embodiment. System 700 includes a probe 701 and a high frequency power supply 728. Probe 701 includes a return electrode 718 and an active electrode assembly/aspiration unit 730. Active electrode assembly/aspiration unit 730 and return electrode 718 are independently coupled to power supply 728. Active electrode assembly/aspiration unit 730 comprises a plurality of elements adapted for ablating and/or modifying tissue at a surgical site, and for removing unwanted materials from the surgical site in an aspiration stream (see, e.g., Fig. 16). Active electrode assembly/aspiration unit 730 is coupled to a suitable vacuum source 770. Vacuum sources, such as vacuum pumps, are well known in the art.

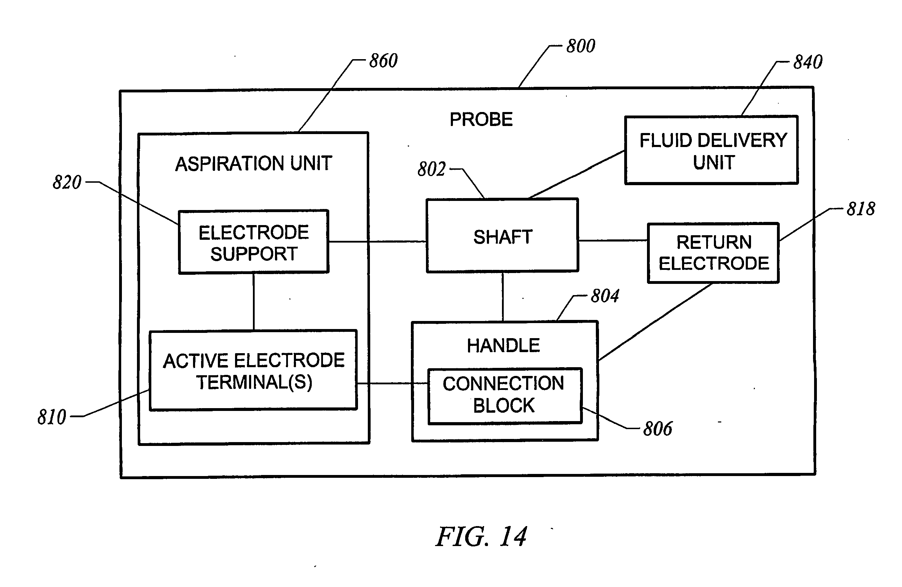

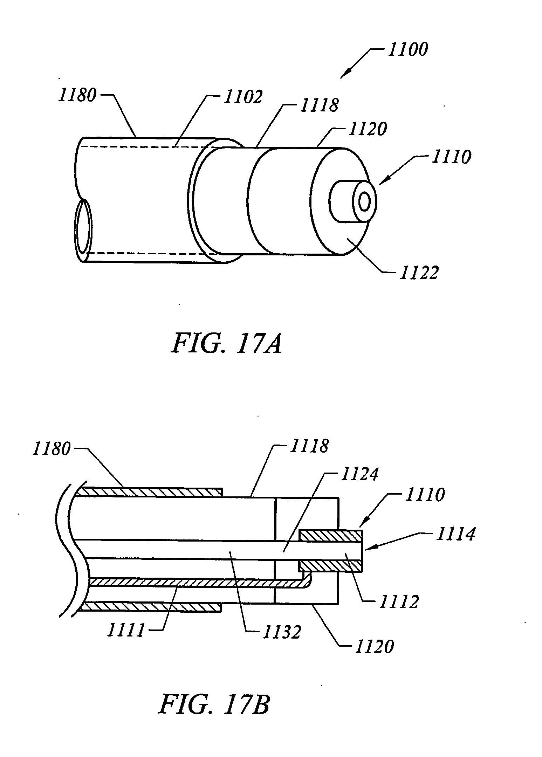

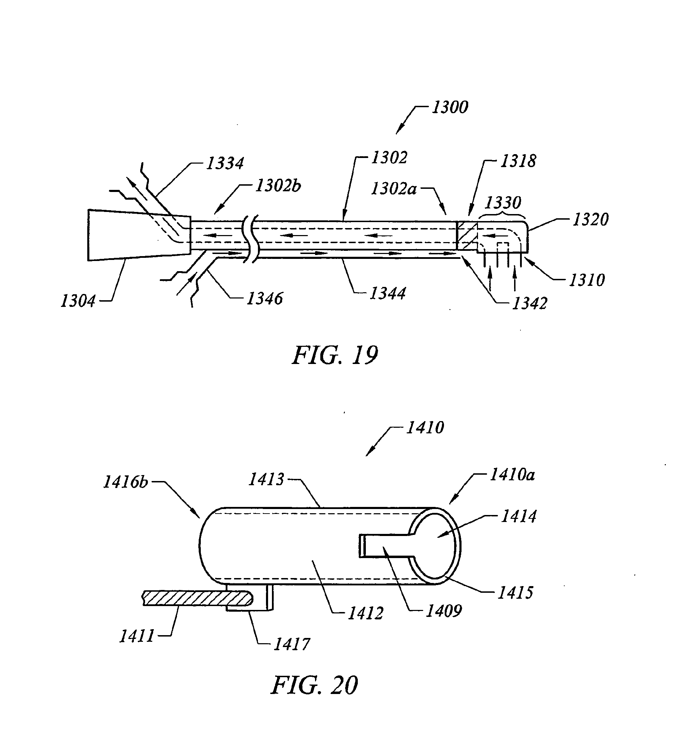

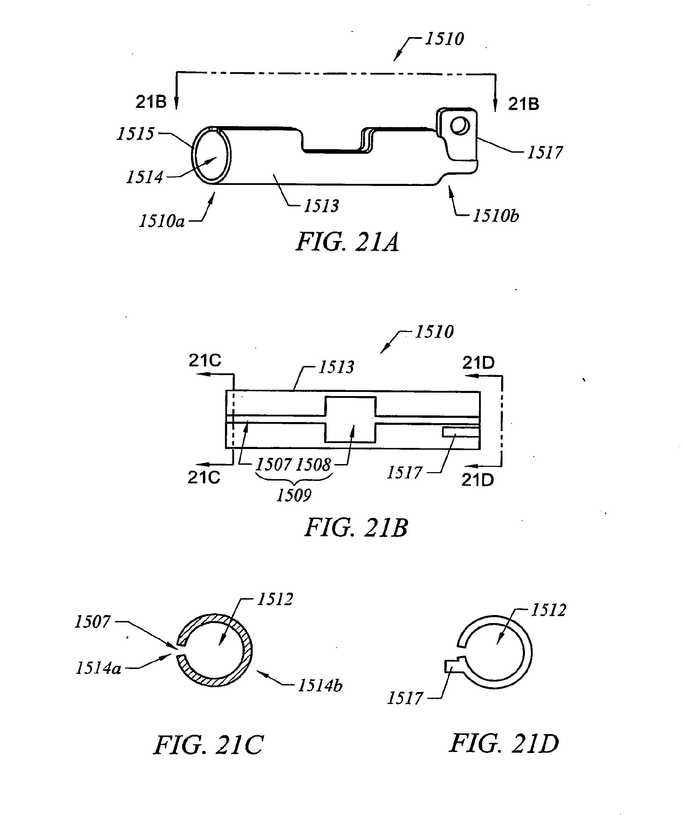

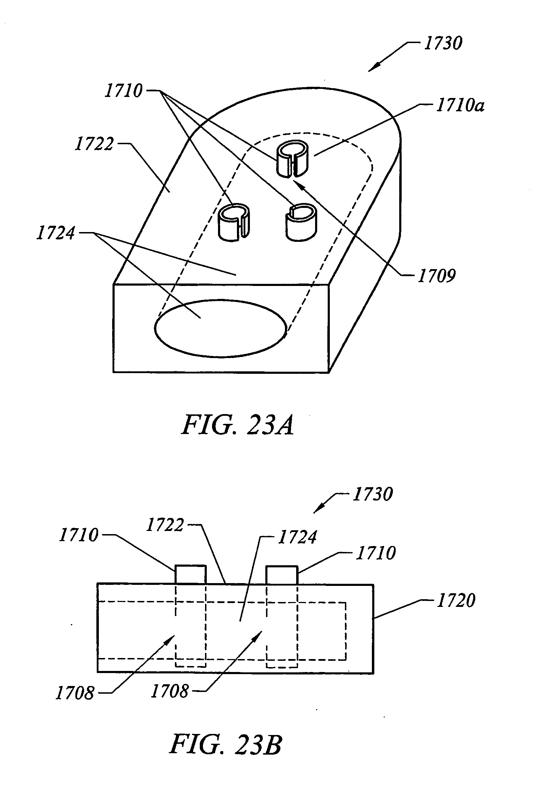

Fig. 14 is a block diagram representing an electrosurgical probe 800, Probe 800 includes a shaft 802, and a handle 804 housing a connection block 806. Probe 800 further includes an aspiration unit 860, and a return electrode 818. Aspiration unit 860 includes an electrically insulating electrode support 820 and at least one active electrode terminal 810. Electrode support 820 may be disposed on shaft 802 either laterally or terminally. Typically, active electrode terminal(s) 810 are arranged within electrode support 820 such that an electrode lumen of each electrode terminal 810 is in communication with a suction cavity within electrode support 820 (e.g., Figs. 17A-B). In some embodiments, a suction opening within each active electrode terminal is in communication with the electrode lumen and with the suction cavity (e.g., Figs. 21A-D, 23). Active electrode terminal(s) 810 and return electrode 818 are independently coupled to connection block 806. Connection block 806 is adapted for connecting active electrode terminal(s) 810 and return electrode 818 to a high frequency power supply (e.g., Figs. 1, 13). Probe 800 still further includes a fluid delivery unit 840 adapted for delivering an electrically conductive fluid to an electrode assembly (e.g., Fig. 19), or to a target tissue at a surgical site. Fluid delivery unit 840 may comprise a fluid delivery lumen located either internal or external to a probe shaft (e.g., Fig. 19).

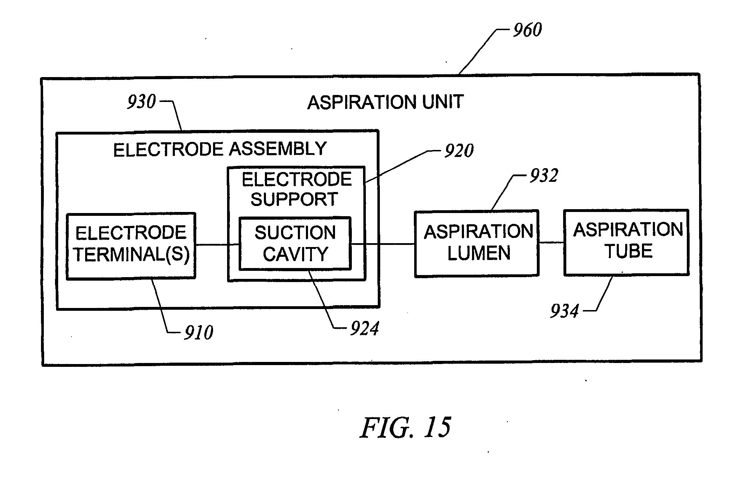

Fig. 15 is a block diagram representing an aspiration unit 960 of an electrosurgical probe. Aspiration unit 960 includes an electrode assembly 930 comprising an electrode support 920 having a suction cavity 924 therein. Electrode assembly 930 further comprises at least one active electrode terminal 910 arranged within electrode support 920, such that a lumen or opening of each electrode terminal 910 is in communication with suction cavity 924. Aspiration unit 960 further includes an aspiration lumen 932 coupled to suction cavity 924, and an aspiration tube 934 coupled to aspiration lumen 932. Unwanted materials may be aspirated from a surgical site via the electrode lumen of each active electrode terminal 910 upon application of suction to aspiration tube 934, e.g., via connection of aspiration tube 934 to a suitable vacuum source. Unwanted materials may include, without limitation, excess bodily fluids, resected tissue fragments, or excess extraneously added electrically conductive fluid.

Fig. 16 is a block diagram representing an electrode assembly 1030 for an electrosurgical probe according to the invention. Electrode assembly 1030 includes at least one active electrode terminal 1010 coupled to an electrically insulating electrode support 1020. In one embodiment, each active electrode terminal 1010 has an electrode lumen 1012 and a suction opening in communication with electrode lumen 1012. Electrode support 1020 includes a suction cavity 1024, and active electrode terminal(s) 1010 are arranged within electrode support 1020 such that electrode lumen 1012 and/or suction opening 1008 are in communication with suction cavity 1024. As an example, in one embodiment, each active electrode terminal comprises a substantially cylindrical body including a wall defining an internal lumen, wherein the suction opening comprises a slit in the wall (e.g., Figs. 21A-D). The body of each electrode terminal is mounted in the electrode support such that the lumen and slit are in communication with the suction cavity of the electrode support. Each active electrode terminal may comprise a metal such as molybdenum, platinum, tungsten, palladium, iridium, titanium, or their alloys.

Fig. 17A is a perspective view of the distal portion of an electrosurgical probe 1100. Probe 1100 includes a shaft 1102 having an electrically insulating electrode support 1120 disposed at the shaft distal end. Electrode support 1120 includes a treatment surface 1122. An active electrode terminal 1110 protrudes from treatment surface 1122. Shaft 1102 may comprise an electrically conducting material, and is partially encased within an electrically insulating sleeve 1180. Such an electrically insulating sleeve may comprise a plastic material, such as a polyester. An exposed (non-insulated) distal portion of shaft 1102 defines a return electrode 1118. Fig. 17B is a side view of the distal portion of probe 1100 of Fig. 17A, showing an electrode lumen 1112 within electrode terminal 1110. Electrode lumen 1112 terminates at its distal end in an electrode port 1114. Electrode lumen 1112 is in communication proximally with a suction cavity 1124 within electrode support 1120. An aspiration lumen 1132 is coupled to suction cavity 1124. Aspiration lumen 1132 is connected to a vacuum source, for example, via an aspiration tube (see, e.g., Figs. 13, 15, 18A). As a result, unwanted materials may be removed from a surgical site via electrode port 1114, electrode lumen 1112, and suction cavity 1124. An active electrode lead 1111 is coupled to electrode terminal 1100.

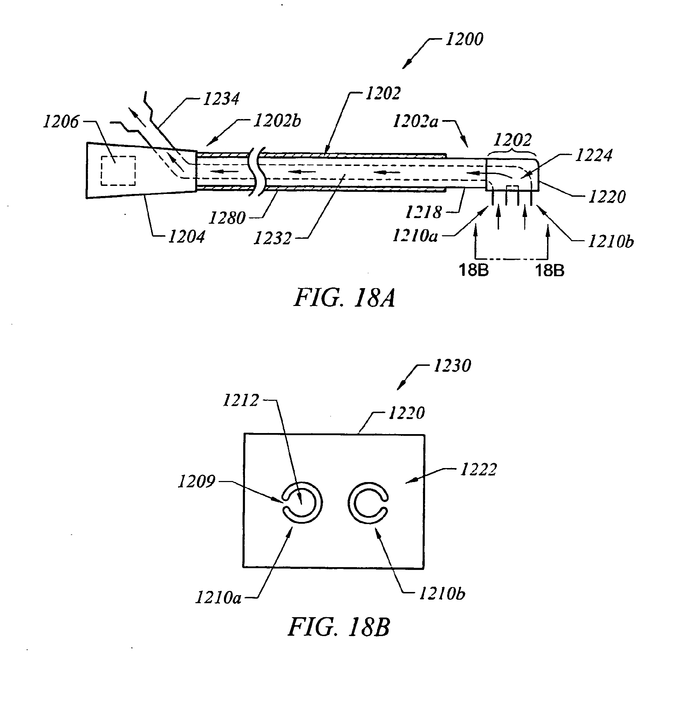

Fig. 18A is side view of an electrosurgical probe 1200, according to the invention. Probe 1200 includes a shaft 1202 having a shaft distal end 1202a and a shaft proximal end 1202b. An electrode assembly 1230 is disposed at shaft distal end 1202a. Electrode assembly 1230 includes an electrically insulating electrode support 1220 having a suction cavity 1224 therein. A plurality of active electrode terminals, represented as 1210a and 1210b, protrude from a treatment surface 1222 (Fig. 18B) of electrode support 1220.

Probe 1200 further includes an aspiration lumen 1232. A distal portion of aspiration lumen 1232 is in communication with suction cavity 1224. In one embodiment, aspiration lumen 1232 lies within shaft 1202, and the distal portion of aspiration lumen 1232 is coupled to a proximal portion of electrode support 1220. However, other arrangements and configurations for an aspiration lumen are possible under the invention. Aspiration lumen 1232 is coupled at its proximal end to an aspiration tube 1234. Aspiration tube 1234 is adapted for connection to a vacuum source, the latter well known in the art. An aspiration stream, indicated by open arrows, extends proximally from electrode terminals 1210a, 1210b to aspiration tube 1234 via aspiration lumen 1232. Unwanted materials removed from a surgical site via the aspiration stream include bodily fluids, such as blood; excess extraneously added electrically conductive fluid; gaseous ablation by-products; and resected tissue fragments. Again with reference to Fig. 18A, shaft 1202 is partially encased within an electrically insulating sleeve 1280. In one embodiment, shaft 1202 is a metal tube comprising a material such as stainless steel, molybdenum, platinum, tungsten, palladium, iridium, titanium, or their alloys. An exposed (non-encased) distal portion of shaft 1202 defines a return electrode 1218. Shaft distal end 1202b is affixed to a handle 1204. Typically, handle 1204 houses a connection block 1206. Return electrode 1218 and active electrode terminals 12120a, 1210b are coupled to connection block 1206. Connection block 1206 is adapted for electrically connecting active electrode terminals 1210a, 1210b and return electrode 1218 to a high frequency power supply (e.g., Fig. 1, Fig. 13).

Fig. 18B is an inferior view of electrode assembly 1230 taken along the lines 18B-18B of Fig. 18A, showing electrode terminals 1210a and 1210b in relation to treatment surface 1222. Each electrode terminal 1210a, 1210b has an electrode lumen 1212 and a suction opening 1209 leading to lumen 1212. According to the present invention each electrode terminal comprises a substantially cylindrical wall, and the suction opening takes the form of a longitudinal slit in the wall (e.g., Figs. 20, 21A-D). Although two electrode terminals are shown in Fig. 18B, other numbers and arrangements of electrode terminals on electrode support 1220 are also within the scope of the invention.

Fig. 19 is a side view of an electrosurgical probe 1300, according to the invention. Probe 1300 includes a number of elements analogous to those of probe 1200 (Fig. 18A), for example, a shaft 1302 having a shaft distal end 1302a and a shaft proximal end 1302b, an aspiration lumen 1332 within shaft 1302, a handle 1304, an electrode assembly 1330 disposed at shaft distal end 1302a, and a return electrode located proximal to electrode assembly 1330. Electrode assembly 1330 includes an electrically insulating electrode support 1320 having a plurality of active electrode terminals 1310 protruding from a treatment surface of electrode support 1320. A distal end of aspiration lumen 1332 is in communication with electrode terminals 1310 via electrode support 1320. Probe 1300 further includes a fluid delivery unit comprising a fluid delivery lumen 1344 coupled at its proximal end to a fluid delivery tube 1346, and terminating at its distal end in a fluid delivery port 1342. Fluid delivery lumen 1344 delivers electrically conductive fluid (represented by solid arrows) to electrode terminals 1310, and/or to a target tissue, via fluid delivery port 1342. The electrically conductive fluid (e.g., isotonic saline) may be delivered so as to provide a current flow path between active electrode terminals 1310 and return electrode 1318. In an alternative embodiment (not shown), a fluid delivery lumen may be coupled to an electrode support, the latter having one or more fluid delivery ports therein. An electrically insulating sleeve (e.g., Fig. 18A), is omitted from Fig. 19 for the sake of clarity.

Fig. 20 is a perspective view of an active electrode terminal 1410, according to the invention. Electrode terminal 1410 includes a body 1413 having a substantially cylindrical wall 1415 defining an electrode lumen 1412. Electrode terminal 1410 has a working end 1410a and a second end 1410b. Electrode lumen 1412 terminates at working end 1410a in an electrode port 1414. Electrode port 1414 is continuous with a suction opening 1409. As shown, suction opening 1409 is in the form of a slit in wall 1415 extending from working end 1410a, however other configurations for suction opening 1409 are also within the scope of the invention (e.g., Figs. 21A-D). Electrode port 1414, electrode lumen 1412, and suction opening 1409 represent a distal portion of an aspiration unit for an electrosurgical probe according to one aspect of the instant invention. In use, working end 1410a protrudes from a treatment surface of an electrode support, and unwanted materials may be drawn through electrode lumen 1412 via port 1414 and/or suction opening 1409 in an aspiration stream. In the embodiment of Fig. 20, second end 1410b is typically open, and allows passage of unwanted materials from a surgical site via the aspiration stream toward downstream components of the aspiration unit, such as a suction cavity and an aspiration lumen (e.g., Figs. 15, 17B, 18A). A flange 1417 located at second end 1410b allows a wire or active electrode lead 1411 to be coupled to electrode terminal 1410. Flange 1417 may also serve to stabilize terminal 1410 within an electrode support.

Figs. 21A-D illustrate an active electrode terminal 1510, according to another embodiment of the invention. Fig. 21A is a perspective view of active electrode terminal 1510. Electrode terminal 1510 includes a body 1513 having a substantially cylindrical wall 1515 defining an electrode lumen 1512 (see, e.g., Figs. 21C-D). Electrode terminal 1510 has a working end 1510a and a second end 1510b. Electrode lumen 1512 terminates at working end 1510a in an electrode port 1514. Perhaps as best seen in Fig. 21B, electrode port 1514 is continuous with a suction opening 1509. Suction opening 1509 comprises a slit 1507 arranged longitudinally in wall 1515, and a window 1508. Window 1508 is in the form of an expanded portion of slit 1507. However other configurations for suction opening 1509 are also within the scope of the invention. Window 1508 serves to increase the area of suction opening 1509, thereby facilitating removal of unwanted materials from a surgical site via an aspiration stream. In use, terminal 1510 is arranged within an electrode support having a suction cavity therein, such that window 1508 is in communication with the suction cavity (e.g., Fig. 16, Fig. 23B). Working end 1510a protrudes from a treatment surface of the electrode support, and unwanted materials may be drawn through electrode lumen 1512 to window 1508, and thence to an aspiration lumen connected to the suction cavity of the electrode support (e.g., Figs. 15, 16).

As seen in Fig. 21C, slit 1507 is located at a first region 1514a of working end 1510a. A second region 1514b lies substantially opposite first region 1514a. The presence of slit 1507 at first region 1514a of working end 1510a results in preferential flow of an aspiration stream at first region 1514a, and results in a relatively shielded zone at second region 1514b. Relatively low flow rate of the aspiration stream at second region 1514b promotes generation and maintenance of a plasma at working end 1510a upon application of a suitable high frequency voltage to electrode terminal 1510 in the presence of an electrically conductive fluid. Facile generation and maintenance of a plasma at working end 1510a is conducive to the efficient and controlled ablation or modification of a target tissue using an electrosurgical apparatus according to one aspect of the instant invention.

Again with reference to Figs. 21A-D, a flange 1517 extends from second end 1510b. Flange 1517 may include a void, e.g., a circular hole therethrough, and facilitates connection of an electrode lead to electrode terminal 1510. In the embodiment of Figs. 21A-D, second end 1510b is typically closed, in which case the aspiration stream flows through window 1508 to the suction cavity within the electrode support. In one embodiment, second end 1510b is closed and terminates within the electrode support (e.g., Fig. 23B). In another embodiment (not shown), second end 1510b may terminate external to the electrode support, and may be closed or sealed with a suitable biocompatible adhesive or sealant.

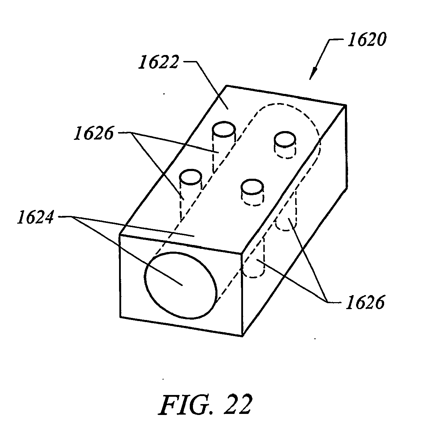

Fig. 22 is a perspective view of an electrode support 1620 for an electrosurgical probe, according to one embodiment of the invention. Electrode support 1620 has a treatment surface 1622. In use, active electrode terminals (e.g., Figs 20, 21A-D) protrude from treatment surface 1622. Electrode support 1620 includes a suction cavity 1624 and a plurality of electrode sockets 1626. As shown, suction cavity 1624 is oriented substantially axially within electrode support 1620, while electrode sockets 1626 are arranged substantially orthogonal to treatment surface 1622 and intersect suction cavity 1624. Each electrode socket 1626 is adapted for accommodating an electrode terminal (e.g., terminals 1410, 1510 of Figs 20, 21A-D, respectively.) The electrode terminals are arranged within electrode support 1620 such that a suction opening (e.g., a slit and/or window, Figs. 21A-D) of each electrode terminal is in communication with suction cavity 1624.

Fig. 23A is a perspective view, and Fig. 23B is a side view, of an electrode assembly 1730, according to another embodiment of the invention. Electrode assembly 1730 comprises an electrode support 1720 and a plurality of active electrode terminals 1710. A suction cavity 1724 is located within support 1720. Typically, each electrode terminal 1710 occupies an electrode socket (e.g., Fig. 22) within electrode support 1720. A working end 1710a of each electrode terminal 1710 protrudes from a treatment surface 1722 of electrode support 1720. A suction opening 1709 extends longitudinally in each electrode terminal 1710 from working end 1710a towards suction cavity 1724. With reference to Fig. 23B, each suction opening 1709 is in communication with suction cavity 1724 via a region 1708 of suction opening 1709. As an example, region 1708 may be in the form of a window or expanded portion of suction opening 1709 (e.g., Figs. 21A-D). Suction opening 1709 and suction cavity 1724 define a portion of an aspiration stream for removing unwanted materials from a surgical site. The plurality of electrode terminals 1710 may be arranged within electrode support 1720 such that suction opening 1709 of each electrode terminal is oriented in a particular direction. As shown in Fig. 23A, three electrode terminals are arranged such that suction opening 1709 of each electrode terminal 1710 is oriented substantially toward the center of treatment surface 1722. However, other configurations for electrode terminals 1710 are also possible under the invention. Similarly, electrode assemblies having other numbers of electrode terminals 1710 are also within the scope of the invention.

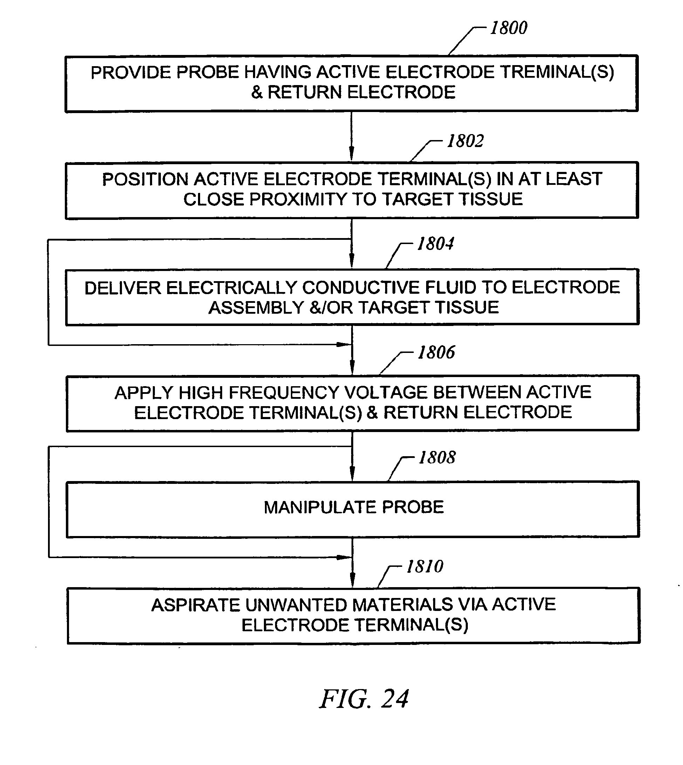

Fig. 24 schematically represents a number of steps involved in a method of treating a target tissue of a patient, using an electrosurgical probe, according to another embodiment of the invention, wherein step 1800 involves providing an electrosurgical probe having at least one active electrode terminal and a return electrode. The probe provided in step 1800 may have elements, features, or characteristics of the various electrosurgical probes described hereinabove (e.g., as described with reference to Figs. 13-23B). In one embodiment, the probe provided in step 1800 includes an electrode assembly having at least one active electrode terminal, each electrode terminal arranged in an electrode socket of an electrically insulating electrode support. Typically, the electrode support includes a suction cavity coupled proximally to an aspiration lumen, and each active electrode terminal has an electrode lumen terminating in an electrode port, and a suction opening in communication with the electrode lumen, wherein a portion of the suction opening is aligned with the suction cavity, and the electrode lumen defines a portion of an aspiration stream by which unwanted materials may be removed from a surgical site during use of the probe.

The electrode support includes a treatment surface, and the active electrode terminal(s) have a working end protruding from the treatment surface. Typically, the probe further includes a shaft having a shaft distal end and a shaft proximal end, the electrode assembly disposed at the shaft distal end, and a handle affixed to the shaft proximal end, the handle housing a connection block. In one embodiment, the shaft comprises a metal tube having a proximal portion encased within an electrically insulating sleeve, and an exposed distal portion defining the return electrode. The connection block is adapted for coupling the active electrode terminals and the return electrode to a high frequency power supply (e.g., Fig. 1). The high frequency power supply is typically adapted for operation in at least one of the ablation mode and the sub-ablation mode (as described hereinabove).

Step 1802 involves positioning the working end of at least one active electrode terminal of the electrode assembly in at least close proximity to the target tissue. In one embodiment, each active electrode terminal has a substantially cylindrical body and a suction opening located at a first region of the working end, the suction opening defining a region of relatively high flow rate of an aspiration stream adjacent to the suction opening, and the working end having a second, shielded region characterized by a relatively low flow rate of the aspiration stream, wherein the second region is located substantially opposite the suction opening.

Optional step 1804 involves delivering an electrically conductive fluid, e.g., via a fluid delivery port and fluid delivery lumen, to the electrode assembly. The electrically conductive fluid (e.g., isotonic saline) provides a current flow path between the active electrode and the return electrode. In one embodiment, the fluid delivery lumen is a tube disposed external to the shaft. In some embodiments, the target tissue is immersed in, or surrounded by, an electrically conductive bodily fluid (e.g., blood, synovial fluid), in which case the step of delivering an extraneous electrically conductive fluid may be omitted.

Step 1806 involves applying, via the high frequency power supply, a high frequency voltage between the active electrode terminal(s) and the return electrode, wherein the high frequency voltage is sufficient to ablate, sculpt, cut, coagulate, or otherwise modify the target tissue. The high frequency power supply may be operated in the ablation mode or the sub-ablation mode, as described hereinabove, according to the desired effect on the target tissue, e.g., in the ablation mode for the volumetric removal of tissue, and in the sub-ablation mode for shrinkage or coagulation of tissue. The actual voltage applied in step 1806 will generally be within the ranges cited hereinabove, for example, from about 70 volts RMS to 500 volts RMS in the ablation mode, and from about 10 volts RMS to 90 volts RMS in the sub-ablation mode.

Optional step 1808 involves manipulating the probe/electrode assembly with respect to the target tissue. Typically, the electrode assembly is manipulated in step 1808 by manipulating the probe via the handle. Step 1808 may involve translating the electrode assembly by reciprocating the probe, whereby the target tissue is sculpted, shaped, dissected, or excised. Typically, volumetric removal (ablation) of target tissue according to the invention involves plasma-induced molecular dissociation of target tissue components. Coagulation or other types of modification of tissue may be attained by applying a suitable voltage to the probe (during step 1806, *supra*) while the power supply is operating in the sub-ablation mode.

Step 1810 involves aspirating any excess electrically conductive fluid, or other unwanted materials (such as resected tissue fragments, bodily fluids, gaseous or liquid ablation by-products, and the like) from the surgical site. According to one embodiment of the invention, unwanted materials are removed from the surgical site in an aspiration stream which enters the electrode assembly via the electrode port/electrode lumen and/or suction opening of each active electrode terminal and flows to the suction cavity of the electrode support. The suction cavity is connected to a proximal aspiration lumen. In one embodiment, the aspiration lumen lies within the shaft, and the proximal end of the aspiration lumen is coupled to an aspiration tube at or near the handle (e.g., Figs. 18A, 19). The aspiration tube and aspiration lumen, together with the electrode terminals and suction cavity of the electrode assembly, comprise an aspiration unit of the probe. The aspiration tube may be coupled to a suitable vacuum source for applying suction to the aspiration unit.

It will be apparent to the skilled artisan that the method described with reference to Fig. 24 is not restricted to a specific surgical procedure, nor to any particular type of procedure, but rather is applicable to a broad range of procedures in which controlled ablation, shrinkage, coagulation, or other electrosurgical modification of a target tissue is required. While the exemplary embodiments of the present invention have been described in detail, by way of example and for clarity of understanding, a variety of changes, adaptations, and modifications will be obvious to those of skill in the art. Therefore, the scope of the present invention is limited solely by the appended claims.

## Claims

1. An electrosurgical probe (1200) for treating a target tissue at a surgical site, comprising:
a shaft (1202) having a shaft distal end (1202a) and a shaft proximal end (1202b); and
an electrode assembly (1230) disposed at the shaft distal end (1202b) wherein the electrode assembly includes an electrically insulating electrode support (1220) and at least one active electrode terminal (1210a, 1210b) arranged on a treatment surface (1222) of the electrode support (1220), wherein the at least one active electrode terminal protrudes from the treatment surface (1222) and each of the at least one active electrode terminal has an internal lumen (1212) therethrough, wherein:
the internal lumen (1212) extends through the treatment surface and is adapted for removing unwanted materials from the surgical site;
the at least one active electrode terminal (1210a, 1210b) comprises a substantially cylindrical body (1413) including a wall (1415) defining the internal lumen (1212, 1412), a working end (1410a) having an open electrode port (1114, 1414) in communication with the internal lumen (1412) **characterised in that** the at least one active electrode terminal (1210a, 1210b) further comprises a suction opening (1209, 1404) in the form of a slit longitudinally formed in said wall (1415), the suction opening laterally oriented in the wall (1415) of the body (1413) and extending proximally from a working end (1410a) of the at least one active electrode terminal, wherein the suction opening (1409) is in communication with the internal lumen (1212, 1412), and the internal lumen is in communication with a suction lumen (1232) within the electrode support (1220).

2. The probe of claim 1, wherein-the internal lumen (1212) terminates in an electrode port (1114) at the working end (1410a).

3. The probe of claim 2, wherein the electrode support (1220) includes a suction cavity (1224).

4. The probe of claim 3, wherein the suction opening causes preferential flow of an aspiration stream at a first region of the working end (1410a).

5. The probe of any one of the above claims, further comprising a return electrode (1218) located on the shaft and proximal to the electrode support.

6. The probe of any one of the above claims, further comprising an electrically insulating sleeve (1202) on a proximal portion of the shaft.

7. The probe of any one of the above claims, further comprising a fluid delivery unit (421, 415) for delivering an electrically conductive fluid to the electrode assembly.

8. The probe of any one of the above claims, wherein the electrode lumen is in communication with a vacuum source (770).

## Patentansprüche

1. Elektrochirurgische Sonde (1200) zur Behandlung eines Zielgewebes an einer chirurgischen Eingriffsstelle, die Folgendes umfasst:
einen Schaft (1202) mit einem distalen Ende (1202a) und einem proximalen Ende (1202b), und
eine Elektrodenbaugruppe (1230), die sich am distalen Ende des Schafts (1202a) befindet, wobei die Elektrodenbaugruppe einen elektrisch isolierenden Elektrodenträger (1220) und mindestens einen aktiven Elektrodenanschluss (1210a, 1210b) beinhaltet, der auf einer Behandlungsoberfläche (1222) des Elektrodenträgers (1220) angeordnet ist, wobei mindestens ein aktiver Elektrodenanschluss aus der Behandlungsoberfläche (1222) hervorsteht und der aktive Elektrodenanschluss oder die aktiven Elektrodenanschlüsse von einem inneren Lumen (1212) durchzogen werden, wobei:
sich das innere Lumen (1212) durch die Behandlungsoberfläche erstreckt und zum Entfernen unerwünschten Materials von der chirurgischen Eingriffsstelle geeignet ist;
der mindesten eine aktive Elektrodenanschluss (1210a, 1210b) einen im Wesentlichen zylinderförmigen Körper (1413) einschließlich einer Wand (1415), die das innere Lumen (1212, 1412) begrenzt, und ein Arbeitsteil (1410a) mit einer offenen Elektrodenmündung (1114, 1414), die mit dem inneren Lumen (1412) in Verbindung steht, umfasst, **dadurch gekennzeichnet, dass** der mindestens eine aktive Elektrodenanschluss (1210a, 1210b) weiterhin eine Absaugöffnung (1209, 1409) in der Form eines Schlitzes umfasst, der in Längsrichtung in der Wand (1415) ausgebildet ist, wobei die Absaugöffnung in der Wand (1415) des Körpers (1413) seitlich ausgerichtet ist und sich proximal von einem Arbeitsteil (1410a) des mindestens einen aktiven Elektrodenanschlusses erstreckt und wobei die Absaugöffnung (1409) in Verbindung mit dem inneren Lumen (1212, 1412) steht, und das innere Lumen in Verbindung mit einem Absauglumen (1232) innerhalb des Elektrodenträgers (1220) steht.

2. Sonde nach Anspruch 1, wobei das innere Lumen (1212) in einer Elektrodenmündung (1114) am Arbeitsteil (1410a) endet.

3. Sonde nach Anspruch 2, wobei der Elektrodenträger (1220) einen Absaughohlraum (1224) umfasst.

4. Sonde nach Anspruch 3, wobei die Absaugöffnung einen bevorzugten Fluss eines Aspirationsstroms an einem ersten Bereich des Arbeitsteils (1410a) bewirkt.

5. Sonde nach einem der vorhergehenden Ansprüche, die weiterhin eine Gegenelektrode (1218) umfasst, die sich am Schaft und proximal zum Elektrodenträger befindet.

6. Sonde nach einem der vorhergehenden Ansprüche, die weiterhin eine elektrisch isolierende Hülse (1202) an einem proximalen Teil des Schafts umfasst.

7. Sonde nach einem der vorhergehenden Ansprüche, die weiterhin eine Flüssigkeitsabgabeeinheit (421, 415) umfasst, um eine elektrisch leitfähige Flüssigkeit an die Elektrodenbaugruppe zu liefern.

8. Sonde nach einem der vorhergehenden Ansprüche, wobei das Elektrodenlumen in Verbindung mit einer Vakuumquelle (770) steht.

## Revendications

1. Sonde électrochirurgicale (1200) permettant de traiter un tissu cible à un site chirurgical, comprenant :
un arbre (1202) ayant une extrémité distale d'arbre (1202a) et une extrémité proximale d'arbre (1202b) ; et
un ensemble d'électrode (1230) agencé à l'extrémité distale de l'arbre (1202a), dans lequel l'ensemble d'électrode comprend un support d'électrode électriquement isolant (1220) et au moins un terminal d'électrode active (1210a, 1210b) agencé sur une surface de traitement (1222) du support d'électrode (1220), dans lequel au moins un terminal d'électrode active fait saillie de la surface de traitement (1222) et chacun d'au moins l'un du terminal d'électrode active possède une lumière interne (1212) à l'intérieur de celui-ci, dans lequel :
la lumière interne (1212) s'étend à travers la surface de traitement et elle est adaptée pour l'élimination de matériels indésirables du site chirurgical ;
l'au moins un terminal d'électrode active (1210a, 1210b) comprend un corps sensiblement cylindrique (1413) comprenant une paroi (1415) définissant une lumière interne (1212, 1412), une extrémité de travail (1410a) ayant un port d'électrode ouverte (1114, 1414) en communication avec la lumière interne (1412) **caractérisée en ce que** l'au moins un terminal d'électrode active (1210a, 1210b) comprend également une ouverture d'aspiration (1209, 1409) sous la forme d'une fente longitudinale formée dans ladite paroi (1415), l'ouverture d'aspiration étant orientée latéralement dans la paroi (1415) du corps (1413) et s'étend proximalement de l'extrémité de travail (1410a) de l'au moins un terminal d'électrode active, dans lequel l'ouverture d'aspiration (1409) est en communication avec la lumière interne (1212, 1412), et la lumière interne est en communication avec une lumière d'aspiration (1232) dans le support de l'électrode (1220).

2. Sonde de la revendication 1, dans laquelle la lumière interne (1212) se termine en un port d'électrode (1114) à l'extrémité de travail (1410a).

3. Sonde de la revendication 2, dans laquelle le support d'électrode (1220) comprend une cavité d'aspiration (1224).

4. Sonde de la revendication 3, dans laquelle l'ouverture d'aspiration entraîne un flux préférentiel d'un courant d'aspiration au niveau d'une première région d'une extrémité de travail (1410a).

5. Sonde de l'une quelconque des revendications précédentes, comprenant également une électrode de retour (1218) située sur l'arbre et proche du support de l'électrode.

6. Sonde de l'une quelconque des revendications précédentes, comprenant également une gaine électriquement isolante (1202) sur la partie proximale de l'arbre.

7. Sonde de l'une quelconque des revendications précédentes, comprenant également une unité d'administration de liquide (421, 415) pour délivrer un liquide électriquement conducteur à l'ensemble d'électrode.

8. Sonde de l'une quelconque des revendications précédentes, dans laquelle la lumière de l'électrode est en communication avec une source de vide (770).
